# EUROPEAN PATENT APPLICATION

(11) **EP 4 678 653 A1**
(43) Date of publication of application: **14.01.2026**
(21) Application number: 24766558.1
(22) Date of filing: 01.03.2024
(51) Int. Cl.: C07K 14/14, A61P 31/14, A61K 38/00, A61K 9/16, A61P 3/10

(54) **MUNS FUSION PROTEIN CAPABLE OF FORMING MICROSPHERES AND USES THEREOF**

(30) Priority: 03.03.2023 ES 202330185
(71) Applicant: Universidade de Santiago de Compostela, 15782 Santiago de Compostela a Coruña (ES)
(72) Inventor: LÓPEZ ABELLA, Daniel, 15782 Santiago de Compostela (A CORUÑA) (ES); BARREIRO PIÑEIRO, Natalia, 15782 Santiago de Compostela (A CORUÑA) (ES); LÓPEZ TEIJEIRO, Adrián, 15782 Santiago de Compostela (A CORUÑA) (ES); MARTÍNEZ COSTAS, José Manuel, 15782 Santiago de Compostela (A CORUÑA) (ES)
(74) Representative: ABG Intellectual Property Law, S.L.
(86) International application number: PCT/ES2024/070125
(87) International publication number: WO 2024/184565

(57) **Abstract**

The invention relates to a fusion protein encoding a polypeptide based on the minimal region of the *Orthoreovirus* muNS protein capable of forming microspheres and/or nanospheres, modified to permit the addition of polypeptides at the C-terminal.

## Description

### FIELD OF THE INVENTION

The invention relates to a fusion protein comprising a polypeptide based on the minimal region of the *Orthoreovirus* muNS protein capable of forming microspheres and/or nanospheres, modified to permit the addition of molecules at the C-terminal end thereof.

### BACKGROUND OF THE INVENTION

Avian reoviruses are members of the genus *Orthoreovirus,* one of the 12 genera of the family Reoviridae. These viruses are important bird pathogens and cause significant economic losses in the poultry industry. Avian reoviruses are non-lipid-enveloped viruses that replicate in the cytoplasm of infected cells and have a genome consisting of 10 double-stranded RNA segments surrounded by a double concentric protein shell 85 nm in diameter. The genomic segments are divided into three classes based on their electrophoretic mobility: three of the L (large) class, three of the M (medium) class, and four of the S (small) class. With the exception of the S1 segment, which is tricistronic, all other genes are monocistronic. The genomic segments are transcribed by means of an RNA-dependent polymerase, producing messenger RNAs (mRNAs) with a nucleotide sequence identical to that of the positive strand of the double-stranded RNA segment. Viral mRNAs have a dual function in infected cells: they program the synthesis of viral proteins in ribosomes and serve as a template for the synthesis of the negative strands of the genomic segments.

A new system that uses inclusion formation by the muNS protein of mammalian reoviruses as a platform for detecting protein interactions *in vivo* in mammalian cells has been described (Miller et al., Mol Cell Proteomics., 2007 6 1027-1038) and has also been adapted for use in yeast (Schmitz et al., Nat Methods 2009; 6 500-2). In this system, the target protein is fused to the C-terminal region of muNS so that the fusion generates cytoplasmic inclusions and attracts the target protein ligand to them. In the yeast system, these authors demonstrate that their system is superior to the double hybrid system in terms of the number and type of interactions detected, at least with the proteins tested in said paper. However, this system has several problems, among which the following stand out: i) certain proteins may fold incorrectly when fused with muNS-Mi and lose the capacity to interact with their ligands; ii) some proteins may interfere with the muNS-Mi inclusion formation capacity and either fail to form such inclusions or generate amorphous intracellular aggregates, greatly altering the detection of interactions; iii) the intracellular localization of the target protein or ligand may not be suitable for detection in cytoplasmic inclusions.

Document WO 2011/098652 describes a system that uses the formation of inclusions by the *Orthoreovirus* muNS protein as a platform for protein purification and for the detection of interactions between proteins both *in vivo* and *in vitro.* This platform is based on the minimal region of the avian *Orthoreovirus* muNS protein capable of forming inclusions, which recruits a peptide tag and the proteins bound to that tag to said inclusions. The peptide tag comprises the minimal region of the muNS protein of an *Orthoreovirus* capable of being incorporated into the inclusions formed by the muNS protein.

Therefore, it is necessary to improve the system for the purpose of reducing its complexity and increasing the usefulness of the muNS system.

### SUMMARY OF THE INVENTION

The inventors have developed a system based on the minimal fragment which is capable of forming inclusions of the muNS protein (muNS-Mi), where they have managed to overcome the previously insurmountable obstacle of modifying the C-terminal end of muNS-Mi through the addition of a sortase recognition sequence that does not affect the capacity of muNS-Mi to form inclusions.

In a first aspect, the invention relates to a fusion protein comprising the following components:
(i) a polypeptide comprising a sequence selected from the group consisting of:
   - sequence 448-635 (SEQ ID NO: 1) of the avian *Orthoreovirus* muNS protein,
   - sequence 518-721 (SEQ ID NO: 2) of the mammalian *Orthoreovirus* muNS protein, and
   - a functionally equivalent variant of any of the above with the capacity to form nanospheres and/or microspheres, and
(ii) a polypeptide selected from:
   (a) a polypeptide comprising the sortase recognition motif or the residual part of a sortase recognition motif generated after a sortase-mediated reaction, wherein component (ii) is fused to the carboxyl end of component (i), or
   (b) a polypeptide comprising the sortase acceptor motif, wherein component (ii) is fused to the amino end of the component.

**In** another aspect, the invention relates to a nanosphere or a microsphere comprising a fusion protein according to the invention.

**In** another aspect, the invention relates to a polynucleotide encoding a fusion protein according to the invention.

**In** another aspect, the invention relates to an expression cassette comprising a polynucleotide according to the invention.

**In** another aspect, the invention relates to a vector comprising a polynucleotide according to the invention or an expression cassette according to the invention.

**In** another aspect, the invention relates to a vector composition comprising the vector according to the invention and a second vector comprising a second polynucleotide encoding a polypeptide selected from the group consisting of:
- a polypeptide comprising sequence 477-542 (SEQ ID NO: 25) of the avian *Orthoreovirus* muNS protein,
- a polypeptide comprising sequence 561-622 (SEQ ID NO: 26) of the mammalian *Orthoreovirus* muNS protein,
- a functionally equivalent variant of any of the above which retains the capacity to be incorporated into nanospheres and/or microspheres.

**In** another aspect, the invention relates to a cell comprising a fusion protein according to the invention, a polynucleotide, an expression cassette, a vector, or a vector composition according to the invention.

**In** another aspect, the invention relates to a method for producing a fusion protein according to the invention, hereinafter method I of the invention, which comprises:
(a) expressing in a cell a first polynucleotide encoding said fusion protein,
(b) subjecting said cell to conditions suitable for the formation of nanospheres or microspheres, and
(c) concentrating the nanospheres or microspheres.

In another aspect, the invention relates to a method for producing a protein, hereinafter method II of the invention, which comprises:
(a) expressing in a cell a first polynucleotide encoding a fusion protein according to the invention and a second polynucleotide encoding a second fusion protein comprising components:
   (i) a polypeptide selected from the group consisting of:
      - a polypeptide comprising sequence 477-542 (SEQ ID NO: 25) of the avian *Orthoreovirus* muNS protein,
      - a polypeptide comprising sequence 518-721 (SEQ ID NO: 26) of the mammalian *Orthoreovirus* muNS protein,
      - a functionally equivalent variant of any of the above which retains the capacity to be incorporated into nanospheres and/or microspheres, and
         (ii) a second polypeptide of interest, wherein component (ii) is the protein that is produced;
(b) subjecting said cell to conditions suitable for the formation of nanospheres and/or microspheres, and
(c) concentrating the nanospheres and/or microspheres.

In another aspect, the invention relates to a method for producing a first polypeptide of interest, hereinafter method III of the invention, wherein the method comprises:
(a) producing the fusion protein of the invention by means of method I of the invention, wherein the fusion protein comprises:
   - component (ii)(a) and a component (iii) fused to the amino end of component (i), wherein component (iii) is the polypeptide of interest, and
   - a protease recognition sequence between its components (i) and (iii),
(b) subjecting the nanospheres and/or microspheres to conditions leading to the disintegration thereof, causing the separation of the fusion protein and the nanospheres and/or microspheres,
(c) contacting the product resulting from step (b) with a protease specific for the recognition sequence connecting components (i) and (iii) of the fusion protein under conditions suitable for the proteolysis of said fusion protein, with the subsequent separation of components (i) and (iii) of the fusion protein,
(d) subjecting the product of step (c) to conditions suitable for the formation of the nanospheres and/or microspheres, and
(e) separating the nanospheres and/or microspheres from component (iii).

In another aspect, the invention relates to a protein obtainable according to method **I,** II, or III according to the invention.

In another aspect, the invention relates to a method for producing a nanosphere or microsphere according to the invention, hereinafter method IV of the invention, wherein said method comprises the steps of:
(a) producing the fusion protein by means of method I according to the invention, and
(b) subjecting said cell to conditions suitable for the formation of nanospheres and/or microspheres.

In another aspect, the invention relates to a pharmaceutical composition or immunogenic composition comprising the nanosphere and/or microsphere according to the invention and a pharmaceutically acceptable excipient.

In another aspect, the invention relates to a nanosphere and/or microsphere according to the invention for use in medicine.

In another aspect, the invention relates to a nanosphere or microsphere according to the invention or an immunogenic composition according to the invention for use in the treatment and/or prevention of bluetongue, wherein the fusion proteins comprised in the nanosphere or the microsphere are characterized by comprising at least one of:
- the bluetongue virus 4 (BTV) outer capsid protein 2 (VP2);
- the bluetongue virus 4 (BTV) core protein 7 (VP7);
- the bluetongue virus 4 (BTV) non-structural protein 1 (NS1);
- a protein with a sequence functionally equivalent to any of the above.

In another aspect, the invention relates to a nanosphere or a microsphere according to the invention or an immunogenic composition according to the invention for use in the treatment and/or prevention of African horse sickness, wherein the fusion proteins comprised in the nanosphere or the microsphere are characterized by comprising at least one of:
- the African horse sickness virus (AHSV) non-structural protein 1 (NS1);
- a protein with a sequence functionally equivalent to any of the above.

In another aspect, the invention relates to a nanosphere or a microsphere according to the invention or a pharmaceutical composition according to the invention for use in the treatment and/or prevention of type 1 diabetes, wherein the fusion protein comprised in the nanosphere or microsphere is characterized by comprising the glucose-6-phosphatase 2 protein (IGRP).

In another aspect, the invention relates to a method for inducing type 1 diabetes in an animal model which comprises administering to an animal an effective amount of the nanosphere or of the microsphere according to the invention.

### BRIEF DESCRIPTION OF THE FIGURES

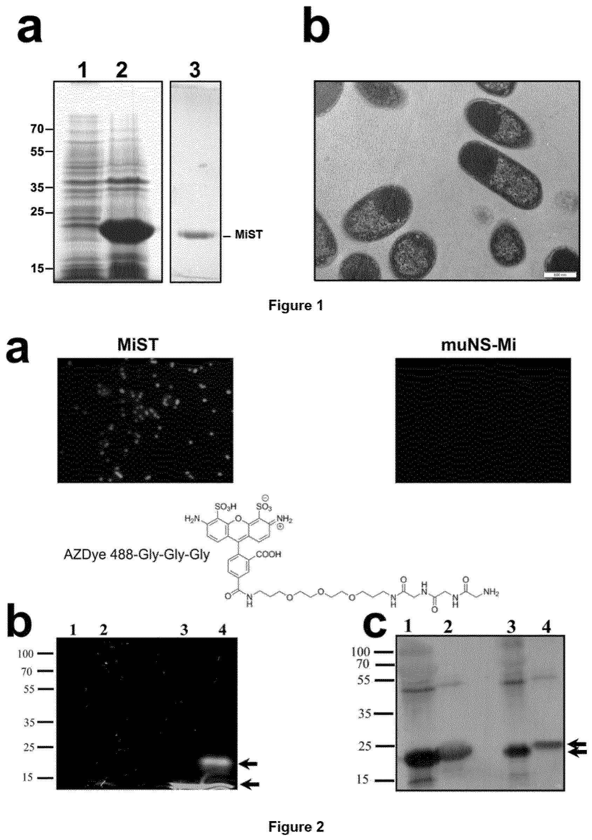

**Figure 1****: Expression of MiST-IC.** A. PAGE analysis of bacteria transformed with pET Duet 1.MiST, either uninduced (1) or induced with IPTG (2). MiST purification is shown in the third lane. MW markers are shown on the left and the theoretical position of MiST on the right of Figure B. A group of the same analyzed bacteria in lane 2a, visualized by means of TEM.
**Figure 2****: Tagging of MiST-IC with AZDye 488-Gly-Gly-Gly.** A. Purified NS obtained after the expression of the proteins indicated in the upper part of each image were incubated with the fluorescent compound AZDye 488-Gly-Gly-Gly (formula below the images) in the presence of sortase A. Excess dye was removed by dialysis, and the NS were observed under a fluorescence microscope. B. Samples of muNS-Mi (1 and 3) or MiST (2 and 4), incubated (3, 4) or not (1, 2) with AZDye 488-Gly-Gly-Gly in the presence of sortase A were resolved by means of PAGE and the unstained and unfixed gel was visualized under UV light. The bands corresponding to fluorescently labeled MiST (4) and to excess fluorescent label (3, 4) are indicated with arrows. C. Samples identical to the samples analyzed in A were subjected to Western blot analysis with anti-muNS antibodies. The positions of MiST and muNS-Mi are indicated with arrows.

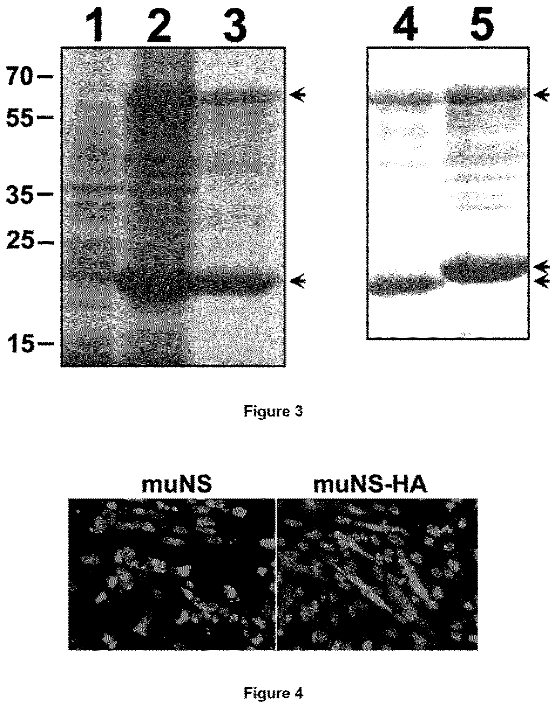

**Figure 3****: Tagging of MiST-IC with AZDye 488-Gly-Gly-Gly.** A. Extracts of bacteria uninduced (1) or induced with IPTG, previously transformed with the dual plasmid expressing AvPAL and either muNS-Mi (2) or MiST (3). 4 and 5 correspond to purified NS of the extracts corresponding to 2 and 3.
**Figure 4****:** HeLa cells were transfected with a plasmid directing the expression of the viral muNS protein (muNS), giving rise to a fusion protein formed by muNS fused at the C-terminal end thereof with the hemagglutinin epitope (muNS-HA). The cells were fixed with paraformaldehyde and analyzed by means of immunofluorescence using anti-muNS antibodies. It can be observed that the addition of HA at the C-terminal end causes the loss in muNS's capacity to form cytoplasmic inclusions.

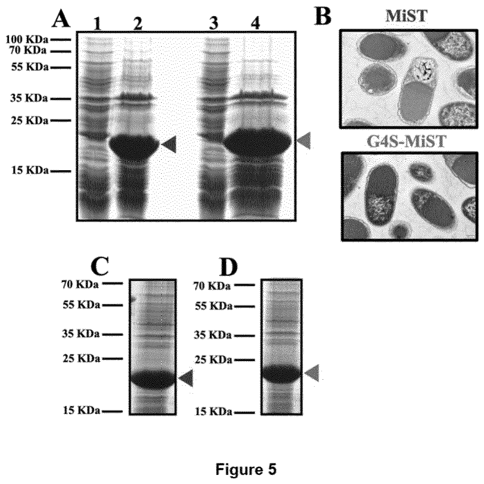

**Figure 5****: Expression and purification of versions of MiST in bacteria. A. Electrophoretic analysis.** SDS-PAGE analysis of bacteria transformed either with pETDuet1. MiST (1, 2) or PETDuet1.G4S-MiST (3, 4), before (1, 3) or after (2, 4) induction with 1 mM IPTG. **B. TEM analysis.** The micrographies of induced bacteria showed the correct formation of both MiST and G4S-MiST NS. **C and D. Electrophoretic analysis of the purification.** The final purification of MiST (C) and G4S-MiST (D) NS is shown. The red and blue arrows show the positions of MiST and G4S-MiST, respectively.

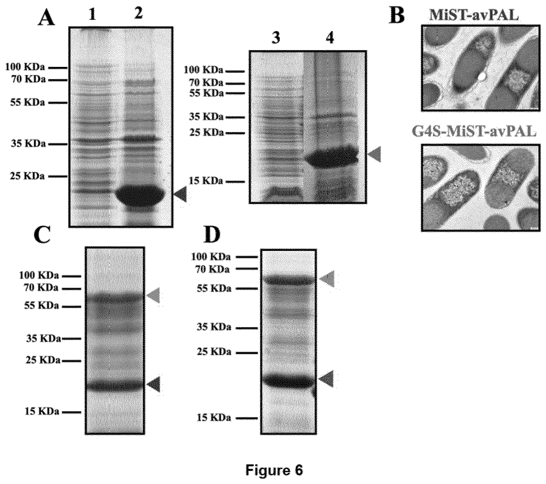

**Figure 6****: Expression and purification of versions of MiST loaded with IC-tagged Av PAL in bacteria. A. Electrophoretic analysis.** SDS-PAGE analysis of bacteria transformed either with pETDuet1.MiST/IC-Av PAL (1, 2) or PETDuet1.G4S-MiST2/IC-Av PAL (3, 4), before (1, 3) or after (2, 4) induction with 1 mM IPTG. **B. TEM analysis.** The micrographies of induced bacteria showed the correct formation of both MiST and G4S-MiST NS. **C and D. Electrophoretic analysis of the purification.** The final purification of MiST (C) and G4S-MiST (D) NS is shown. The red and blue arrows show the positions of MiST and G4S-MiST, respectively.

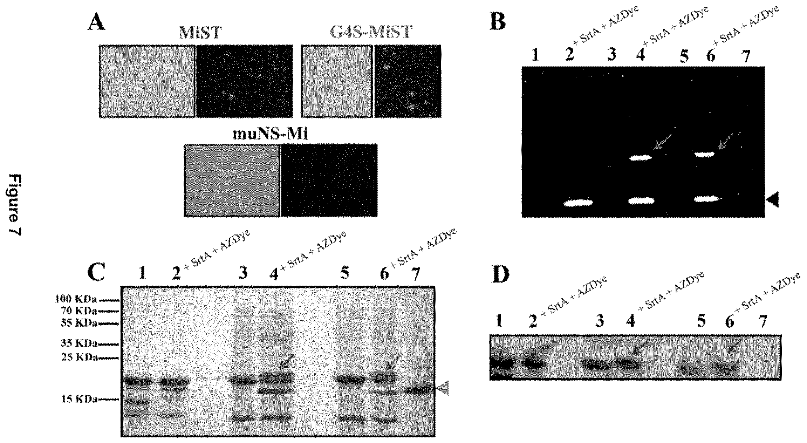

**Figure 7****: SrtA-mediated derivatization of MiST-IC NS. A. Fluorescence microscopy analysis.** Purified NS obtained after the expression of the proteins indicated in the upper part of each image were incubated with the substrate AZDye 488-Gly-Gly-Gly in the presence of SrtA. They were observed with a 100x lens. **B. Fluorescent tagging SDS-PAGE analysis.** NS samples of muNS-Mi (lanes 1 and 2), MiST (lanes 3 and 4), and G4S-MiST (lanes 5 and 6), incubated (2, 4, 6) or unincubated (1, 3, 5) with the mentioned fluorescent substrate in the presence of SrtA, were resolved by means of SDS-PAGE, and the unstained and unfixed gel was visualized under UV light. The fluorescently labeled bands corresponding to MiST and G4S-MiST (lanes 4 and 6) are indicated with a violet arrow. Excess unbound fluorescent compound (lanes 2, 4, and 6) is indicated by means of a black triangle at the front edge. **C. Coomassie staining of proteins separated by SDS-PAGE.** The same polyacrylamide gel as in B was subjected to Coomassie staining. The positions of labeled MiST and G4S-MiST are indicated with a violet arrow. The position of SrtA is indicated with a green triangle. **D. Western blot analysis.** Samples identical to those analyzed in the aforementioned gels were subjected to Western blot analysis using anti-muNS antibodies. Lane 7 corresponds to SrtA samples.

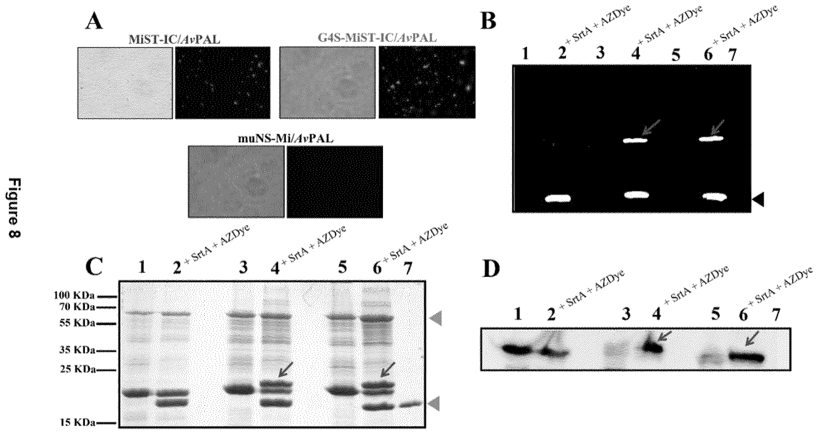

**Figure 8****: SrtA-mediated derivatization of MiST-IC NS loaded with av-PAL.** The same analysis from the preceding figure was repeated with MiST or G4S-MiST nanospheres loaded with AvPAL, obtaining the same results.

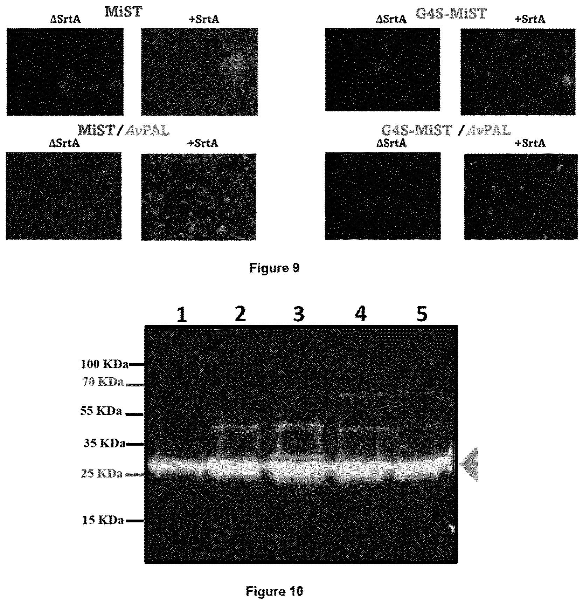

**Figure 9****: Fluorescence microscopy analysis.** Samples of the indicated versions of MiST, loaded or unloaded with AvPAL as indicated, were incubated with eGFP that contained polyglycine and with (Srt+) or without sortase A, as indicated in the upper part of each image.
**Figure 10****: PAGE-UV analysis.** The samples were mixed with Laemmli but not boiled to maintain the semi-denatured conformation ("fluorescent gel") of eGFP. In this case, the band corresponding to the size of eGFP is indicated in all the lanes with the green arrow. Sample 1 is an unreacted eGFP control. And samples 2, 3, 4, and 5 are NS of the different versions of MiST that reacted with eGFP in the presence of sortase A (1:MiST, 2: G4S-MiST, 3: MiST/IC-AvPAL, 4: G4S-MiST/IC-AvPAL).

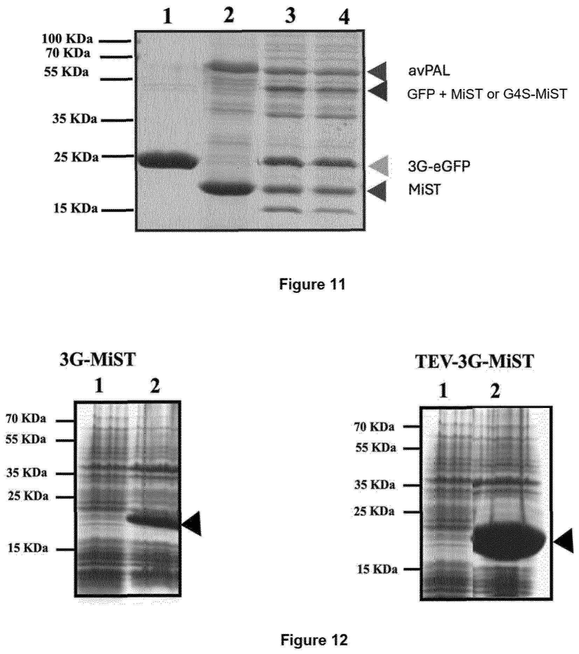

**Figure 11****: SDS-PAGE analysis of Gly-GFP-modified MiST nanospheres.** Lane 1 corresponds to glycine-containing GFP. Lane 2 corresponds to MiST NS loaded with AvPAL. Lanes 3 and 4 are Mist (3) or G4S-MiST (4) NS loaded with AvPAL after the reaction of sortase A in the presence of glycine-containing GFP. The positions of each protein residue are indicated with arrows on the right side of the image.
**Figure 12****: Expression of 3G-MiST and TEV-3G-MiST.** SDS-PAGE analysis of bacteria transformed with plasmid pDuet1-3 G.MiST (left panel) or pDuet1-TEV-3G-MiST (right panel) before (1) or after (2) induction with 1 mM IPTG. The gel was stained with Coomassie blue. Positions of molecular weight markers are indicated on the left in each image.

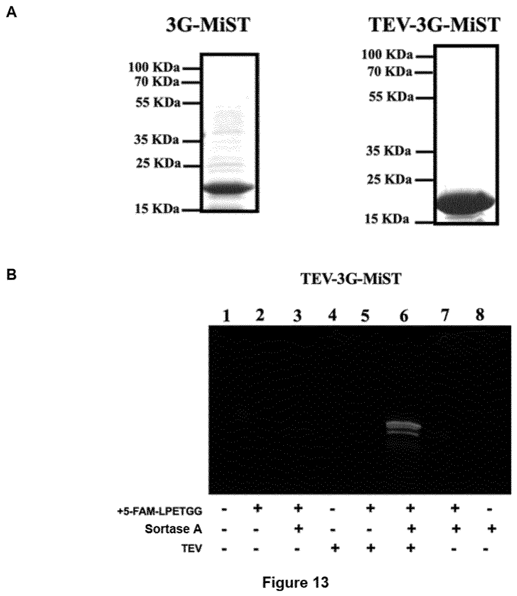

**Figure 13****: Purification and SrtA-mediated derivatization of 3G-MiST and TEV-3G-MiST.** A. Fluorescence microscopy analysis. A: Coomassie-stained SDS-PAGE gels with samples of purified 3G-MiST (left panel) and TEV-3G-MiST (right panel). B. Fluorescent tagging SDS-PAGE analysis. NS samples of TEV-3G-MiSt shown in A were incubated or unincubated, as indicated in the lower part of the figure, with fluorescent substrate 5-FAM-LPETGG, sortase A, or TEV protease. The gel was observed and photographed under ultraviolet light to visualize the covalently bound substrate.
**Figure 4****: Purification and SrtA-mediated derivatization of 3G-MiST and TEV-3G-MiST loaded with AvPAL.** A. Fluorescence microscopy analysis. A: Coomassie-stained SDS-PAGE gels with samples of 3G-MiST (left panel) and TEV-3G-MiST (right panel), both of which are loaded with AvPAL and purified. B. Fluorescent tagging SDS-PAGE analysis. NS samples of TEV-3G-MiSt with AvPAL which are shown in A were incubated or unincubated, as indicated in the lower part of the figure, with fluorescent substrate 5-FAM-LPETGG, sortase A, or TEV protease. The gel was observed and photographed under ultraviolet light to visualize the covalently bound substrate.

### DETAILED DESCRIPTION OF THE INVENTION

The authors of the present invention have shown that it is possible to obtain fusion proteins containing the minimal region of the muNS protein (muNS-Mi), which forms nanospheres/microspheres, and a signal at the C-terminal of muNS-Mi which permits the post-translational modification of muNS-Mi both in the monomer and in aggregates in the form of nanospheres/microspheres.

### Fusion protein of the invention

In that sense, in a first aspect the invention relates to a fusion protein, hereinafter the fusion protein of the invention, comprising the following components:
(i) a polypeptide comprising a sequence selected from the group consisting of:
   - sequence 448-635 (SEQ ID NO: 1) of the avian *Orthoreovirus* muNS protein,
   - sequence 518-721 (SEQ ID NO: 2) of the mammalian *Orthoreovirus* muNS protein, and
   - a functionally equivalent variant of any of the above with the capacity to form nanospheres and/or microspheres, and
(ii) a polypeptide selected from:
   (a) a polypeptide comprising the sortase recognition motif or the residual part of a sortase recognition motif generated after a sortase-mediated reaction, wherein component (ii) is fused to the carboxyl end of component (i), or
   (b) a polypeptide comprising the sortase acceptor motif, wherein component (ii) is fused to the amino end of component (i).

The term "protein", used herein interchangeably with "polypeptide", refers to an amino acid chain of any length, wherein the different amino acids are bound to one another by means of peptide bonds.

The term "fusion protein", as used herein, refers to polypeptides comprising two or more regions originating from different or heterologous proteins. A fusion protein, by definition, is never found in nature as such.

The fusion protein of the invention comprises two components:

### Component (i) - Polypeptide

Component (i) of the fusion protein of the invention is a polypeptide selected from the group consisting of:
- sequence 448-635 (SEQ ID NO: 1) of the avian *Orthoreovirus* muNS protein,
- sequence 518-721 (SEQ ID NO: 2) of the mammalian *Orthoreovirus* muNS protein, and
- a functionally equivalent variant of any of the above with the capacity to form nanospheres and/or microspheres.

In a particular embodiment, the polypeptide that is part of the fusion protein of the invention comprises or consists of sequence 448-635 (SEQ ID NO: 1) of the avian *Orthoreovirus* muNS protein.

The term "avian *Orthoreovirus"* or "avian reovirus", as used herein, refers to one of the twelve genera belonging to the family of *Reoviridae* viruses, and specifically to the group within the genus that infects birds. They have double-stranded RNA genomes and, for that reason, belong to group III viruses.

The term "avian *Orthoreovirus* muNS protein", as used herein, refers to one of the non-structural proteins encoded by the avian reovirus or avian *Orthoreovirus* M3 gene and is the only avian reovirus protein capable of forming nanospheres and/or microspheres when expressed in the absence of other factors (Touris-Otero et al. Virology, 319; 94-1069). It is a protein having 635 amino acids defined by accession number Ay608700 in the NCBI database (version Ay608700.1, August 7, 2004) or by accession number AAS78998 in the NCBI database (version AAS78998.1, April 10, 2006). In a particular embodiment, the avian *Orthoreovirus* muNS protein has the sequence SEQ ID NO: 3.

Sequence 448-635 (SEQ ID NO: 1) of the avian *Orthoreovirus* muNS protein is a fragment of the avian *Orthoreovirus* muNS protein comprising the minimal region of the muNS protein of an *Orthoreovirus* having the capacity to form inclusions when expressed in a cell or a functionally equivalent variant thereof.

The term "inclusion(s)", as used herein, refers to nuclear or cytoplasmic aggregates, normally of proteins. Specifically, the protein forming inclusions in the genus *Orthoreovirus* is the muNS or µNS protein, which is capable of forming inclusions when expressed in the absence of other viral factors (Touris-Otero *et al., supra).* When the inclusions are formed by the muNS-Mi protein, they are small sized and spherical, forming what is referred to as "nanospheres" and/or "microspheres". The nanospheres and microspheres are distinguished by their size (see below). In that sense, in the present invention, the term "inclusions" likewise encompasses the terms "nanosphere" and "microsphere".

The capacity to form inclusions of the avian *Orthoreovirus* muNS protein is determined by the presence of the minimal region of the avian *Orthoreovirus* muNS protein corresponding to residues 448 to 635 of said protein. However, any fragment of the avian *Orthoreovirus* muNS protein containing said minimal fragment is capable of forming said inclusions.

In that sense, in a particular embodiment, component (i) of the fusion protein of the invention comprises the full sequence of the avian *Orthoreovirus* muNS protein, or a fragment of the avian *Orthoreovirus* muNS protein comprised between residues 1 and 635 of said protein, or from residue 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 447 and up to residue 635 consecutive amino acids of the avian *Orthoreovirus* muNS protein. In another particular embodiment, component (i) of the fusion protein of the invention comprises from a fragment from residue 447 and up to residue 636 of the avian *Orthoreovirus* protein, or from residue 447 and up to residue 640, 650, 660, 670, 680, 690, 700, 710, 720, or up to residue 721 of the avian *Orthoreovirus* protein. In another particular embodiment, component (i) of the fusion protein of the invention comprises from a fragment of the avian *Orthoreovirus* muNS protein comprised between residues 10 and 720 of said protein, or between residues 10 and 700, or between residues 20 and 700, or between residues 30 and 700, or between residues 40 and 700, or between residues 60 and 680, or between residues 80 and 660, or between residues 100 and 640, or between residues 120 and 635 of said protein.

In another particular embodiment, the polypeptide that is part of the fusion protein of the invention comprises or consists of sequence 518-721 (SEQ ID NO: 2) of the mammalian *Orthoreovirus* muNS protein.

The term "mammalian *Orthoreovirus",* as used herein, refers to one of the twelve genera belonging to the family of *Reoviridae* viruses and specifically to the group within the genus that infects mammals. They have double-stranded RNA genomes and, for that reason, belong to group III viruses.

The term "mammalian *Orthoreovirus* muNS or µNS protein", as used herein, refers to one of the non-structural proteins encoded by the mammalian reovirus or mammalian *Orthoreovirus* and is the only mammalian reovirus protein capable of forming microspheres when expressed in the absence of other viral factors (Becker, M. M. et al. 2003. J. Virol. 77:5948-5963). It is a protein having 721 amino acids defined by accession number ABP48918 in the NCBI database (version ABP48918.1, April 11, 2008) (SEQ ID NO: 4).

In the preferred embodiment in which the *Orthoreovirus* muNS protein is the mammalian *Orthoreovirus* muNS protein, the minimal region of the mammalian *Orthoreovirus* muNS protein having the capacity to form inclusions when expressed in a cell comprises the region corresponding to residues 518 to 721 (SEQ ID NO: 2). In an even more preferred embodiment, component (i) of the fusion protein of invention comprises the whole protein or from residue 2 and up to residue 721 of said protein, or from residue 5, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 470, 480, 490, 500, 510 and up to residue 721 of said protein.

In a particular embodiment, the polypeptide that is part of the fusion protein of the invention comprises or consists of a functionally equivalent variant of (i) sequence 448-635 (SEQ ID NO: 1) of the avian *Orthoreovirus* muNS protein, or of (ii) sequence 518-721 (SEQ ID NO: 2) of the mammalian *Orthoreovirus* muNS protein, with the capacity to form nanospheres and/or microspheres.

In a particular embodiment, the functionally equivalent variants of sequence 448-635 (SEQ ID NO: 1) of the avian *Orthoreovirus* muNS protein or of sequence 518-721 (SEQ ID NO: 2) of the mammalian *Orthoreovirus* muNS protein retain at least 50%, at least 60%, at least 70%, at least 80%, at least 90%, at least 95%, at least 100% of the capacity to form nanospheres/microspheres of the sequences from which they are derived.

The functionally equivalent variants of sequence 448-635 (SEQ ID NO: 1) of the avian *Orthoreovirus* muNS protein or of sequence 518-721 (SEQ ID NO: 2) of the mammalian *Orthoreovirus* muNS protein include those that show at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

The degree of identity between variants and the sequences from which they are derived is determined using algorithms and computer methods that are well known by those skilled in the art. The identity between two amino acid sequences is preferably determined using the BLASTP algorithm [BLASTManual, Altschul, S., et al., NCBI NLM NIH Bethesda, Md. 20894, Altschul, S. et al., J. Mol Biol, 215: 403 - 410 (1990)]. In a more particular embodiment, the sequence identity between the functionally equivalent variant and the protein sequence from which it is derived is calculated along the entire length of the polypeptide.

"Functionally equivalent variant" is understood to mean all those peptides derived from sequence 448-635 (SEQ ID NO: 1) of the avian *Orthoreovirus* muNS protein or from sequence 518-721 (SEQ ID NO: 2) of the mammalian *Orthoreovirus* muNS protein by means of modification, insertion, substitution, and/or deletion of one or more amino acids, provided that the function of the muNS protein sequences from which they are derived is substantially retained.

Sequence 448-635 (SEQ ID NO: 1) of the avian *Orthoreovirus* muNS protein and sequence 518-721 (SEQ ID NO: 2) of the mammalian *Orthoreovirus* muNS protein have the capacity to form nanospheres and/or microspheres in a cell. The functionally equivalent variants of sequence 448-635 (SEQ ID NO: 1) of the avian *Orthoreovirus* muNS protein and of sequence 518-721 (SEQ ID NO: 2) of the mammalian *Orthoreovirus* muNS protein substantially retain the capacity of said sequences to form nanospheres/microspheres in a cell. Methods suitable for determining the capacity to form nanospheres/microspheres in a cell include, but are not limited to, the method described in Example 1 of patent application WO 2011/098652 based on the expression of the protein of interest in a cell and analysis of the formation of nanospheres/microspheres (inclusions) through indirect immunofluorescence, using polyclonal antibodies against the epitope of interest or an epitope fused to the protein of interest, being able to check for the formation of nanospheres and/or microspheres. In a particular embodiment, the functionally equivalent variants of sequence 448-635 (SEQ ID NO: 1) of the avian *Orthoreovirus* muNS protein or of sequence 518-721 (SEQ ID NO: 2) of the mammalian *Orthoreovirus* muNS protein retain at least 50%, at least 60%, at least 70%, at least 80%, at least 90%, or 100% or more of the capacity of said sequences to form nanospheres/microspheres in a cell.

In a particular embodiment, the functionally equivalent variants of sequence 448-635 (SEQ ID NO: 1) of the avian *Orthoreovirus* muNS protein or of sequence 518-721 (SEQ ID NO: 2) of the mammalian *Orthoreovirus* muNS protein include those that show at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived and retain at least 50%, at least 60%, at least 70%, at least 80%, at least 90%, or 100% or more of the capacity of said sequences to form nanospheres/microspheres in a cell.

As will be apparent to one skilled in the art, it may be useful for component (i) of the fusion protein of the invention to have another polypeptide of interest at its amino end. In that sense, in a particular embodiment, the fusion protein of the invention further comprises a component (iii) fused to the amino end of component (i), where component (iii) is a first polypeptide of interest.

As one skilled in the art will understand, the separation of component (iii) from component (i) may be useful. A possibility to enable separating said components is the use of a peptide the sequence of which contains a cleavage target for a protease, thereby permitting the separation of the two components.

In a particular embodiment, component (iii) is fused to component (i) by a protease recognition sequence. The term "protease" or "peptidases", as used herein, refers to enzymes that break peptide bonds of proteins, using for that purpose a water molecule, so they are classified as hydrolases. Examples of protease cleavage sites suitable for incorporation into the fusion protein of the invention include, without limitation, enterokinase (cleavage site DDDDK; SEQ ID NO: 5), factor Xa (cleavage site IEDGR; SEQ ID NO: 6), thrombin (cleavage site LVPRGS; SEQ ID NO: 7), TEV protease (cleavage site ENLYFQG; SEQ ID NO: 8), PreScission protease (cleavage site LEVLFQGP; SEQ ID NO: 9), inteins, and the like. In a particular embodiment, the protease recognition sequence is an enterokinase recognition sequence or a factor Xa recognition sequence.

In another particular embodiment, component (iii) comprises a secretory pathway signal peptide.

The term "secretory pathway signal peptide", used herein interchangeably with "signal sequence" or "signal peptide" or "localization signal peptide", refers to a short peptide (5-30 amino acids long) present at the N-terminal end which directs the transport of proteins destined for the secretory pathway, whether they are proteins residing in certain organelles (ER, Golgi apparatus, or endosomes), proteins secreted by the cell, or proteins inserted in the cell membrane. The signal peptide directs the translocation of the protein to which it is bound to the ER. During or after translocation, the signal peptide is cleaved by a signal peptidase, generating a free signal peptide and a mature protein.

Non-limiting examples of secretory pathway signal peptides include the signal peptides appearing in class I and II major histocompatibility complex molecules, cytokine or immunoglobulin signal sequences, invariant chain signal sequences, or Lampl, Tapasin, Erp57, Calreticulin, and Calnexin proteins. In a particular embodiment, the secretory pathway targeting sequence is selected from the group consisting of:
- sequence MGWSLILLFLVAVATGVHSQ (SEQ ID NO: 10);
- sequence MMSFVSLLLVGILFWATEAEQLTKCEVFQ (SEQ ID NO: 11);
- human PTH1R signal peptide (MGTARIAPGLALLLCCPVLSSAYAL, SEQ ID NO: 12);
- human cytochrome c oxidase VIII mitochondrial localization sequence (MSVLTPLLLRGLTGSARRLPVPRAK, SEQ ID NO: 13)
- human mGluR5 signal peptide (MVLLLILSVLLLKEDVRGSA, SEQ ID NO: 14);
- human GABAB2R signal peptide (MASPRSSGQPGPPPPPPPPPARLLLLLLLPLLLPLAPG, SEQ ID NO: 15);
- human calreticulin signal peptide (MLLSVPLLLGLLGLAVA, SEQ ID NO: 16); and
- human Igγ2b heavy chain signal peptide, (MGWSCIILFLVATATGKGLTVAGLRSGHIYG, SEQ ID NO: 17).

In a preferred embodiment, the secretory pathway signal peptide comprises sequence MGWSLILLFLVAVATGVHSQ (SEQ ID NO: 10).

When the fusion protein comprises a signal peptide and travels through the secretory pathways, the absence of glycosylations permits a more efficient formation of inclusions in said secretory pathways.

In a particular embodiment, component (i) of the fusion protein of the invention comprises a secretory pathway signal peptide and does not contain N-glycosylations. Therefore, in a particular embodiment, component (i) lacks N-glycosylation consensus sequences. The term "N-glycosylation consensus sequence", as used herein, refers to the sequence made up of - Asn-X-Ser/Thr, wherein X is not proline, which is the most representative consensus sequence, as well as the least abundant N-glycosylation consensus sequences, such as sequences -Asn-Gly- , -Asn-X-Cys, and -Asn-X-Val.

In a preferred embodiment, component (i) comprises a secretory pathway signal peptide and the sequence SEQ ID NO: 1 or a functionally equivalent variant thereof, wherein the amino acid in position 57 is not an Asn residue. In an even more preferred embodiment, component (i) comprises the sequence SEQ ID NO: 18.

In another preferred embodiment, component (i) comprises a secretory pathway signal peptide and the sequence SEQ ID NO: 2 or a functionally equivalent variant thereof, wherein the amino acid in position 57 is not an Asn residue. In an even more preferred embodiment, the second component comprises the sequence SEQ ID NO: 19.

In another preferred embodiment, component (i) comprises a secretory pathway signal peptide and the sequence SEQ ID NO: 2 or a functionally equivalent variant thereof, wherein the amino acid in position 113 is not an Asn residue. In an even more preferred embodiment, the second component comprises the sequence SEQ ID NO: 20.

In another preferred embodiment, component (i) comprises a secretory pathway signal peptide and the sequence SEQ ID NO: 2 or a functionally equivalent variant thereof, wherein the amino acids in positions 57 and 113 are not Asn residues. In an even more preferred embodiment, the second component comprises the sequence SEQ ID NO: 21.

As will be apparent to one skilled in the art, it may be desirable for the fusion protein of the invention to further contain a tag to facilitate purification thereof.

Therefore, in another particular embodiment, component (i) and/or component (iii) comprises a peptide to facilitate purification thereof. In another particular embodiment, component (i) comprises a peptide to facilitate purification thereof, wherein said peptide is fused at the amino end of component (i).

The term "peptide to facilitate purification", as used herein, refers to a peptide useful for isolating or purifying component (iii) or the fusion protein of the invention. In that sense, said peptide is capable of binding one or more ligands from an affinity matrix, such as affinity chromatography. An example of said peptide is the histidine tail (His-tag) which may contain six histidine residues (His6 or H6), which can bind to a nickel or cobalt column with high affinity. Other examples of said peptides include, but are not limited to, Arg-tag, FLAG-tag, Strep-tag, an epitope capable of being recognized by an antibody, such as c-myc-tag (recognized by an anti-c-myc antibody), SBP-tag, S-tag, calmodulin-binding peptide, cellulose-binding domain, chitin-binding domain, glutathione S-transferase-tag, maltose-binding protein, NusA, TrxA, DsbA, Avi-tag, etc. One skilled in the art will understand that peptides to facilitate purification are also useful for detecting the polypeptide to which they are bound. This can be carried out by means of conventional techniques, for example, techniques based on antibodies that specifically recognize the peptide to facilitate purification.

The peptide to facilitate purification thereof can be in the N- or C-terminal position with respect to component (iii). In a preferred embodiment, the peptide to facilitate purification thereof is in the N-terminal position with respect to component (iii). In another preferred embodiment, the peptide to facilitate purification thereof is in the C-terminal position with respect to component (iii).

### Component (ii)(a) - polypeptide comprising the sortase recognition motif or the residual part of said motif

As described, the fusion protein is made up of at least two components, where the second can be a polypeptide comprising the sortase recognition motif.

The term "sortase", or "sortase enzyme", as used herein, refers to a prokaryotic enzyme having a catalytic domain with activity capable of selectively cleaving a peptide bond of a polypeptide chain in the sortase recognition motif and catalyzing a transpeptidation reaction, which gives rise to the formation of an amide bond between the terminal carboxyl group created by the cleavage and a cell wall surface protein in a cell. Sortases are present in almost all Gram-positive bacteria, as well as in some Gram-negative bacteria and Archaea.

Sortases are classified in four different classes (A, B, C, D). Each of the sortase classes cleaves a different recognition motif, and some members of the class cleave multiple peptide motifs. Class A sortase typically cleaves recognition motif LPXTG (SEQ ID NO: 22), wherein X represents any amino acid, between threonine and glycine. The cleaved peptide retains the glycine residue at its amino end, and the cleaved protein binds to the peptidoglycan in the threonine residue. In a particular embodiment, the sortase is a sortase A, an enzymatically active fragment, or a variant or derivative thereof. In another particular embodiment, sortase A is the *Staphylococcus aureus* wild-type sortase, evolved sortase (eSrtA), eSrtA(2A-9), eSrtA(4S-9), or *Streptococcus pyogenes* sortase A. In a particular embodiment, the sortase is S. *aureus* sortase. In a particular embodiment, the sortase recognition motif is amino acid sequence LPXTG (SEQ ID NO: 22), wherein X is any amino acid.

The term "sortase recognition motif", as used herein, refers to a small amino acid sequence (3 to 7 amino acids), recognized by the sortase enzyme and where the transpeptidation reaction is performed. This sequence is normally located at the C-terminal of the protein. In another particular embodiment, the sortase recognition motif is amino acid sequence LPETG (SEQ ID NO: 23).

As one skilled in the art will understand, in certain cases it is possible that the interaction of the sortase recognition motif, the sortase, and the sortase acceptor motif present in a component of interest, is favored through the increase in the flexibility of the polypeptide comprising the sortase recognition motif. In that sense, in a particular embodiment component (i) and component (ii)(a), comprising the sortase recognition motif, are covalently bound through a flexible peptide linker.

The term "flexible peptide linker", as used herein, refers to spacer amino acid sequences which can act as a hinge region between two polypeptide components, permitting them to move independently of one another while maintaining the three-dimensional shape of individual domains, such that the presence of peptide linkers or spacers does not alter the functionality of any of components (i) and (ii)(a). In that sense, a preferred flexible peptide linker according to the invention would be a hinge region characterized by a structural ductility that permits this movement. The effect of the linking region is to provide space between components (i) and (ii)(a). It is thereby ensured on one hand that the secondary and tertiary structures of component (i) or (ii)(a) are not affected by the presence of any of the other components, and that the sortase recognition motif, the sortase, and the component with the sortase acceptor motif interact more easily. In a particular embodiment, the flexible peptide linker preferably comprises at least 2 amino acids, at least 3 amino acids, at least 5 amino acids, at least 10 amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids, or about 100 amino acids.

In another particular embodiment, the flexible peptide linker comprises at least 2 amino acids selected from a group consisting of: serine, glycine, alanine, and threonine. In another particular embodiment, the flexible peptide linker is a polyglycine sequence. In another particular embodiment, the flexible peptide linker comprises a sequence selected from the group consisting of: GGGGS (SEQ ID NO: 29), SGGTSGSTSGTGST (SEQ ID NO: 30), AGSSTGSSTGPGSTT (SEQ ID NO: 31), GGSGGAP (SEQ ID NO: 32), GGGVEGGG (SEQ ID NO: 33), PKPSTPPGSS (SEQ ID NO: 34), AAA, and AAALE (SEQ ID NO: 35). In another particular embodiment, the flexible peptide linker comprises sequence GGGGS (SEQ ID NO: 29).

The presence of component (ii)(a) in the fusion protein of the invention permits the modification of the protein through the covalent binding of any molecule containing a sortase acceptor motif. In a particular embodiment, the fusion protein further comprises a component of interest covalently bound to the sortase recognition motif or to the residual part of a sortase recognition motif generated after a sortase-mediated reaction.

As is apparent to one skilled in the art, the modification of the sortase recognition motif by means of a sortase-mediated reaction, produces a "residual part of a sortase recognition motif", wherein the fusion protein of the invention is modified by means of cleavage of the sortase recognition motif, due to the cleavage of one or more amino acids from the C-terminal of the sortase recognition motif sequence. In that sense, when the sortase recognition motif is amino acid sequence LPXTG (SEQ ID NO: 22), wherein X is any amino acid, the residual part of said motif is LPXT, given that it is the part that remains after the proteolytic cleavage by the sortase of the carboxyl-terminal glycine.

In another particular embodiment, the component of interest is characterized by comprising a sortase acceptor motif.

The term "sortase acceptor motif", as used herein, refers to a polypeptide sequence that acts as an acceptor for the sortase-mediated transfer of a polypeptide to the sortase recognition motif. In a particular embodiment, the sortase acceptor motif is located at, near, adjacent to, or towards the N- or C-terminal end of the component of interest and comprises a non-polar amino acid sequence, where said sequence is at least one amino acid long. In yet another particular embodiment, the non-polar amino acid sequence comprises at least between 1 and 20 amino acids (for example, 1, 2, 3, 4, 5, 6, 7, 8, 9, 10, 11, 12, 13, 14, 15, 16, 17, 18, 19, 20 amino acids or any interval thereof) long. In certain embodiments, the non-polar amino acid sequence is or comprises one or a plurality of glycines or alanines. Exemplary sortase acceptor motifs include G[G]n, where n=0-5 and A[A]n, where n=0-5.

In a particular embodiment, the component of interest is characterized by comprising a sortase acceptor motif, wherein the sortase acceptor motif is characterized by comprising an oligoglycine sequence of at least 3 glycines.

In a particular embodiment, the component of interest is selected from a group consisting of: toll-like receptor (TLR) ligands, monophosphoryl lipid A (MPL A), oligonucleotides selected from CpG islands and polyinosinic:polycytidylic acid (poly(I:C) - CAS number 24939-03-5), saponin, lipid molecules, coating molecules, oligosaccharides, antibodies, nanoantibodies, affitins, viral antigen, a bacterial antigen, a fungal antigen, an allergen, a cell-penetrating peptide, an affibody, or an environmental antigen, a tumor antigen, wherein the component of interest is characterized by comprising an oligoglycine sequence of at least 3 glycines. Non-limiting examples of components of interest would be TLR (toll-like receptor) ligands, MPL (monophosphoryl lipid A), oligonucleotides (CpG, poly(I:C), etc.), saponin, lipid molecules, other lipids, coating molecules for various purposes, such as polyglycerols, polyethylene glycol, chitosans, poly(oxazolines) (POX), poly(hydroxypropyl methacrylate) (PHPMA), poly(2-hydroxyethyl methacrylate) (PHEMA), poly(N-(2-hydroxypropyl)methacrylamide) (HPMA), polyvinylpyrrolidone (PVP), poly(N,N-dimethylacrylamide) (PDMA), poly(N-acryloylmorpholine) (PAcM), etc., oligosaccharides, molecules that direct the spheres *in vivo* to different targets such as nanobodies, affitins, and antibodies. As will be apparent to one skilled in the art, any molecule containing an oligoglycine of at least 3 glycines having a free N-terminal may be coupled.

As will be apparent to one skilled in the art, the viral antigen, bacterial antigen, fungal antigen, allergen, cell-penetrating peptide, affibody, environmental antigen, tumor antigen defined above are likewise valid as components of interest, wherein the component of interest comprises an oligoglycine sequence of at least 3 glycines.

### Component (ii)(b) - polypeptide comprising the sortase acceptor motif

As described, the fusion protein is formed by at least two components, where one of the possibilities for the second component is a polypeptide comprising the sortase acceptor motif described above.

**In** yet another particular embodiment, the sortase acceptor motif comprises a non-polar amino acid sequence, wherein the non-polar amino acid sequence comprises at least between 1 and 20 amino acids (for example, 1, 2, 3, 4, 5, 6, 7, 8, 9, 10, 11, 12, 13, 14, 15, 16, 17, 18, 19, 20 amino acids or any interval thereof) long. **In** certain particular embodiments, the non-polar amino acid sequence is or comprises one or a plurality of glycines or alanines. In another particular embodiment, the sortase acceptor motif comprises a sequence according to G[G]n, where n=0-2, and A[A]n, where n=0-2.

**In** a particular embodiment, component (ii)(b) is characterized by comprising a sortase acceptor motif, wherein the sortase acceptor motif is characterized by comprising an oligoglycine sequence of at least 1 glycine (G). In another particular embodiment, the sortase acceptor motif comprises an amino acid sequence selected from a group consisting of: G, A, GG, AA, GGG, and AAA. **In** another particular embodiment, the sortase acceptor motif is characterized by consisting of an oligoglycine sequence of 3 glycines (GGG). In another particular embodiment, the sortase acceptor motif is characterized by not comprising an oligoglycine sequence of more than 3 glycines. In another particular embodiment, the sortase acceptor motif is characterized by not comprising an oligoglycine sequence of 5 glycines.

**In** certain cases, in order to stabilize the production of the fusion protein, it may be necessary to protect the sortase acceptor motif at the N-terminal end with a polypeptide sequence that can then be cleaved.

**In** that sense, in a particular embodiment, the fusion protein further comprises a protease recognition sequence covalently bound to the amino end of the sortase acceptor motif. In a particular embodiment, the protease recognition sequence is an enterokinase recognition sequence or a factor Xa recognition sequence. The term "protease recognition sequence" has been defined above, and said definitions and particular embodiments are likewise valid for the present embodiments.

In a particular embodiment, the fusion protein comprising component (ii)(b) further comprises a component of interest covalently bound to the sortase acceptor motif.

In a particular embodiment, the component of interest is characterized by comprising a sortase recognition motif. In a particular embodiment, the sortase is a sortase A, an enzymatically active fragment, or a variant or derivative thereof. In another particular embodiment, sortase A is the *Staphylococcus aureus* wild-type sortase, evolved sortase (eSrtA), eSrtA(2A-9), eSrtA(4S-9), or *Streptococcus pyogenes* sortase A. In a particular embodiment, the sortase is S. *aureus* sortase. In a particular embodiment, the sortase recognition motif is amino acid sequence LPXTG (SEQ ID NO: 22), wherein X is any amino acid. In another particular embodiment, the sortase recognition motif is amino acid sequence LPETG (SEQ ID NO: 23).

In another particular embodiment, the component of interest is selected from a group consisting of: toll-like receptor (TLR) ligands, monophosphoryl lipid A (MPL A), oligonucleotides selected from CpG islands and polyinosinic:polycytidylic acid (poly(I:C) - CAS number 24939-03-5), saponin, lipid molecules, coating molecules, oligosaccharides, antibodies, nanoantibodies, affitins, viral antigen, a bacterial antigen, a fungal antigen, an allergen, a cell-penetrating peptide, an affibody, or an environmental antigen, a tumor antigen, wherein the component of interest is characterized by comprising a sortase recognition sequence or the residual part of a sortase recognition motif generated after a sortase-mediated reaction.

### Nanospheres and/or microspheres of the invention

As mentioned above, the fusion protein of the invention has the capacity to form inclusions when expressed in a cell. Said inclusions may vary in size and shape and are referred to as nanospheres or microspheres.

In that sense, another aspect of the present invention relates to a nanosphere or a microsphere comprising at least one fusion protein of the invention, hereinafter the nanosphere/microsphere of the invention.

All the definitions and particular embodiments described in relation to other aspects of the invention are likewise valid and applicable to the present aspect.

As described above, the nanospheres and microspheres have different sizes. In that sense, in a particular embodiment the nanosphere has a size between 300 nanometers (nm) and 550 nm. In another particular embodiment, the microsphere has a size between 0.55 micrometers (µm) and 4 µm, preferably between 1 µm and 4 µm.

The structure of the minimal region of the *Orthoreovirus* muNS protein comprises two coil domain regions at the N- and C-terminal ends, C1 and C2, and an interdomain region referred to as intercoil (IC). This domain corresponds to sequence 477-542 (SEQ ID NO: 25) of the avian *Orthoreovirus* muNS protein or to sequence 561-622 of the mammalian *Orthoreovirus* muNS protein (SEQ ID NO: 26). Said fragment has the capacity to be incorporated into nanospheres and microspheres formed by the *Orthoreovirus* muNS protein.

In that sense, in a particular embodiment, the nanosphere/microsphere of the invention comprises a second fusion protein comprising the following components:
(a) a polypeptide selected from the group consisting of:
   - a polypeptide comprising sequence 477-542 (SEQ ID NO: 25) of the avian *Orthoreovirus* muNS protein,
   - a polypeptide comprising sequence 561-622 (SEQ ID NO: 26) of the mammalian *Orthoreovirus* muNS protein,
   - a functionally equivalent variant of any of the above which retains the capacity to be incorporated into nanospheres and/or microspheres, and
(b) a second polypeptide of interest.

"Functionally equivalent variant" is understood to mean all those peptides derived from sequence 477-542 (SEQ ID NO: 25) of the avian *Orthoreovirus* muNS protein or from sequence 561-622 (SEQ ID NO: 26) of the mammalian *Orthoreovirus* muNS protein by means of modification, insertion, substitution, and/or deletion of one or more amino acids, provided that the function of the muNS protein sequences from which they are derived is substantially retained.

Sequence 477-542 (SEQ ID NO: 25) of the avian *Orthoreovirus* muNS protein and sequence 561-622 (SEQ ID NO26X) of the mammalian *Orthoreovirus* muNS protein have the capacity to be incorporated into microspheres/nanospheres formed by the fusion protein of the invention in a cell. The functionally equivalent variants of sequence 477-542 (SEQ ID NO: 25) of the avian *Orthoreovirus* muNS protein and sequence 561-622 (SEQ ID NO: 26) of the mammalian *Orthoreovirus* muNS protein substantially retain the capacity of said sequences to be incorporated into the microspheres/nanospheres formed by the fusion protein of the invention in a cell. Methods suitable for determining the capacity to be incorporated into the microspheres/nanospheres include, but are not limited to, the method described in Example 3 of patent application WO 2011/098652 based on the formation of the nanospheres/microspheres (inclusions) and expression of the protein of interest in the form of a fusion protein associated with the fragments directing it to the nanospheres/microspheres. Then, indirect immunofluorescence will be carried out using polyclonal antibodies against the HA epitope or the epitope of interest, being able to check for the incorporation of said fragments into the nanospheres/microspheres.

The functionally equivalent variants of sequence 477-542 (SEQ ID NO: 25) of the avian *Orthoreovirus* muNS protein or of sequence 561-622 (SEQ ID NO: 26) of the mammalian *Orthoreovirus* muNS protein have the capacity to be incorporated into microspheres and/or nanospheres. In a particular embodiment, the functionally equivalent variants of sequence 477-542 (SEQ ID NO: 25) of the avian *Orthoreovirus* muNS protein or of sequence 561-622 (SEQ ID NO: 26) of the mammalian *Orthoreovirus* muNS protein retain at least 50%, at least 60%, at least 70%, at least 80%, at least 90%, at least 95%, at least 100% of the capacity to be incorporated into nanospheres and/or microspheres of the sequences from which they are derived.

The functionally equivalent variants of sequence 477-542 (SEQ ID NO: 25) of the avian *Orthoreovirus* muNS protein or of sequence 561-622 (SEQ ID NO: 26) of the mammalian *Orthoreovirus* muNS protein include those that show at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

In a particular embodiment, the functionally equivalent variants of sequence 477-542 (SEQ ID NO: 25) of the avian *Orthoreovirus* muNS protein or of sequence 561-622 (SEQ ID NO: 26) of the mammalian *Orthoreovirus* muNS protein retain at least 50%, at least 60%, at least 70%, at least 80%, at least 90%, at least 95%, at least 100% of the capacity to be incorporated into nanospheres and/or microspheres of the sequences from which they are derived and show at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

The two components (a) and (b) of the second fusion protein can be connected in any order, i.e., component (b) can be fused to the amino terminal end of component (a) or component (a) can be fused to the amino terminal end of component (b). In a particular embodiment, component (b) is fused to the amino terminal end of component (a).

The term "polypeptide of interest", as used herein, refers to any one polypeptide, of any size, that is of interest for the user, wherein the fusion to the amino end of component (i) does not affect the capacity of the fusion protein of the invention to form nanospheres and/or microspheres. In a preferred embodiment, said polypeptide of interest can be a viral antigen, a bacterial antigen, a fungal antigen, an allergen, a cell-penetrating peptide, an affibody, or an environmental antigen or a tumor antigen.

Viral antigens suitable as the first polypeptide of interest include HIV-1 antigens (such as tat, nef, gp120 or gp160, gp40, p24, gag, env, vif, vpr, vpu, rev), human herpes virus antigens (such as gH, gL, gM, gB, gC, gK, gE, or gD or derivatives thereof) or immediate early protein antigens such as SHV1 or SHV2 ICP27, ICP47, ICP4, ICP36, cytomegalovirus antigens, particularly human cytomegalovirus antigens (such as gB or derivatives thereof), Epstein Barr virus antigens (such as gp350 or derivatives thereof), varicella-zoster virus antigens (such as gpl, II, III, and IE63), or a hepatitis virus such as hepatitis B virus antigens (for example hepatitis B surface antigen or hepatitis core antigen), hepatitis C virus antigens (for example core antigens, E1, NS3 or NS5), paramyxovirus antigens such as respiratory syncytial virus antigens (such as F and G proteins or derivatives thereof), parainfluenza virus antigens, rubeola virus antigens (such as EI and E2 proteins), measles virus antigens, human papillomavirus antigens (for example HPV6, 11, 16, 18, e.g., LI, L2, EI, E2, E3, E4, E5, E6, E7), flavivirus antigens (for example yellow fever virus, dengue virus, tick-borne encephalitis virus, Japanese encephalitis virus antigens) or cells infected with virus influenza, such as HA, NP, NA, or M proteins, or combinations thereof), rotavirus antigens (such as VP7sc and other rotavirus components), and the like.

Bacterial antigens suitable as the first polypeptide of interest include *Neisseria spp,* including *N. gonorrhea* and *N. meningitidis* antigens (transferrin-binding proteins, lactoferrin-binding proteins, PiIC, and adhesins); S. *pyogenes* antigens (such as M proteins or fragments thereof and protease C5A); S. *agalactiae, S. mutans; H. ducreyi; Moraxella spp,* including M *catarrhalis,* also known as *Branhamella catarrhalis,* antigens (such as high and low molecular weight adhesins and invasins); *Bordetella spp,* including *B. pertussis,* for example *Parapertussis* and B. *bronchiseptica* antigens (such as pertactin, pertussis toxin, or derivatives thereof, filamentous hemagglutinin, adenylate cyclase, fimbriae); *Mycobacterium spp.,* including *M. tuberculosis, M. bovis, M. leprae, M. avium, M. paratuberculosis, M. smegmatis* antigens; *Legionella spp,* including *L. pneumophila,* antigens (for example ESAT6, antigen 85A, -B or -C, MPT 44, MPT59, MPT45, HSPIO, HSP65, HSP70, HSP 75, HSP90, 19 kDa PPD [Rv3763], 38 kDa PPD [Rv0934]); *Escherichia spp,* including enterotoxigenic *E. coli* antigens (for example colonization factors, heat-labile toxin or derivatives thereof, heat-stable toxin or derivatives thereof), enterohemorrhagic *E. coli* and enteropathogenic *E. coli* antigens (for example Shiga toxin-like toxins or derivatives thereof); *Vibrio spp,* including *V. cholera,* antigens (for example cholera toxin or derivatives thereof); *Shigella spp,* including S. *sonnei, S. dysenteriae, S. flexnerii* antigens; *Yersinia spp,* including *Y. enterocolitica,* antigens (for example a Yop protein); *Y. pestis, Y. pseudotuberculosis; Campylobacter spp,* including *C*. *jejuni* antigens (for example toxins, adhesins, and invasins); *Salmonella spp,* including *S. typhi, S. paratyphi, S. choleraesuis,* S. *enteritidis* antigens; *Listeria spp.,* including *L. monocytogenes* antigens; *Helicobacter spp,* including *H. pylori* antigens (for example urease, catalase, vacuolating toxin); *Pseudomonas spp,* including *P. aeruginosa* antigens; *Staphylococcus spp.,* including S. *aureus, S. epidermidis* antigens; *Enterococcus spp.,* including *E. faecalis, E. faecium* antigens; *Clostridium spp.,* including C. *tetani* antigens (for example tetanus toxin and derivative thereof); *C. botulinum* antigens (for example botulinum toxin and derivative thereof), *C. difficile* antigens (for example clostridium A or B toxins and derivatives thereof); *Bacillus spp.,* including B. *anthracis* antigens (for example anthrax toxin and derivatives thereof); *Corynebacterium spp.,* including *C. diphtheriae* antigens (for example diphtheria toxin and derivatives thereof); *Borrelia spp.,* including B. *burgdorferi* antigens (for example OspA, OspC, DbpA, DbpB); *B. garinii* antigens (for example OspA, OspC, DbpA, DbpB), *B. afzelii* antigens (for example OspA, OspC, DbpA, DbpB), *B. andersonfi* antigens (for example OspA, OspC, DbpA, DbpB), B. *hermsii; Ehrlichia spp.,* including *E. equi* antigens and the agent of human granulocytic ehrlichiosis; *Rickettsia spp,* including R. *rickettsii* antigens; *Chlamydia spp.,* including *C. trachomatis* antigens (for example MOMP, heparin-binding proteins); *Chlamydia pneumoniae* antigens (for example MOMP, heparin-binding proteins), *C. psittaci* antigens; *Leptospira spp.,* including *L. interrogans* antigens; *Treponema spp.,* including *T. pallidum* antigens (for example rare outer membrane proteins), *T. denticola, T. hyodysenteriae* antigens; *Toxoplasma spp.* and *T. gondii* antigens (for example SAG2, SAGS, Tg34); M. *tuberculosis* antigens (such as Rv2557, Rv2558, RPFs: Rv0837c, Rv1884c, Rv2389c, Rv2450, Rv1009, aceA (Rv0467), PstS1, (Rv0932), SodA (Rv3846), 16 kDa Rv2031c, Tb Ra12, Tb H9, Tb Ra35, Tb38-1, Erd 14, DPV, MTI, MSL, mTTC2 and hTCC1); Chlamydia antigens (such as high molecular weight protein (HMWP), ORF3 (document EP 366 412) and possible membrana proteins (Pmp); *Streptococcus spp,* including *S. pneumoniae* antigens (PsaA, PspA, streptolysin, choline-binding proteins, pneumolysin protein antigen, and detoxified mutant derivatives thereof); antigens derived from *Haemophilus spp.,* including H. *influenzae* type B (for example PRP and conjugates thereof); non-typeable *H. influenzae* antigens (such as OMP26, high molecular weight adhesins, P5, P6, protein D, and lipoprotein D, and fimbrin and fimbrin-derived peptides, or multi-copy variants or the fusion proteins thereof).

Fungal antigens suitable as the first polypeptide of interest include, but are not limited to, for example, Candida fungal antigen components; Histoplasma fungal antigens such as heat shock protein 60 (HSP60) and other Histoplasma fungal antigen components; *Pneumocystis spp.,* including *P. carinii* fungal antigens; cryptococci fungal antigens such as capsular polysaccharides and other cryptococci fungal antigen components; coccidia fungal antigens such as spherule antigens and other coccidia fungal antigen components; *Candida spp.,* including *C. albicans* antigens; *Cryptococcus spp.,* including *C. neoformans* antigens; and Tinea fungal antigens such as trichophytin and other coccidia fungal antigen components.

Protozoan antigens suitable as the first polypeptide of interest include, but are not limited to, *Plasmodium spp.,* such as *P. falciparum* antigens and antigens derived from *Plasmodium falciparum* (such as RTS.S, TRAP, MSP1, AMA1, MSP3, EBA, GLURP, RAP1, RAP2, sequestrin, PfEMP1, Pf332, LSA1, LSA3, STARP, SALSA, PfEXP1, Pfs25, Pfs28, PFS27/25, Pfs16, Pfs48/45, Pfs230, and the analogues thereof in *Plasmodium spp.);* as well as merozoite surface antigens, sporozoite surface antigens, circumsporozoite antigens, gametocyte/gamete surface antigens, pf blood stage antigen, 55/RESA, and other plasmoid antigen components; Toxoplasma antigens such as SAG-I, p30, and other Toxoplasma antigen components; schistosome antigens such as glutathione-S-transferase, paramyosin, and other schistosome antigen components; *Trichomonas spp.,* including *T. vaginalis* antigen; *Entamoeba spp.,* including E. *histolytica* antigens; *Babesia spp.,* including B. *microti* antigens; Leishmania antigen and other Leishmania antigens such as gp63, lipophosphoglycan and the associated protein thereof and other Leishmania antigen components; Giardia spp., including *G. lamblia* antigens; and *Trypanosoma cruzi* antigens such as the 75-77 kDa antigen, the 56 kDa antigen, and other Trypanosoma antigen components.

Environmental antigens or allergens suitable as a first polypeptide of interest include, but are not limited to, an antigen derived from naturally occurring allergens such as pollen allergens (tree, ragweed, weed, and grass pollen allergens), insect allergens (inhalable, saliva, and venom allergens), animal dander and hair allergens, and food allergens. Important tree, grass, and ragweed pollen allergens originate from taxonomic orders of Fagales, Oleales, Pinaceae, and Platanaceae, including, among others, birch (Betula), alder (Alnus), hazel (Corylus), hornbeam (Carpinus), and olive (Olea), cedar (Cryptomeria and Juniperus), banana (Platanus), the order Poales including, among others, grasses of the genera Lolium, Phleum, Poa, Cynodon, Dactylis, Holcus, Phalaris, Secale, and Sorghum, the orders Asterales and Urticales including, among others, ragweeds of the genera Ambrosia, Artemisia, and Parietaria. Other allergenic antigens that may be used include allergens of house dust mites of the genera Dermatophagoides and Euroglyphus, storage mites for example Lepidoglyphys, Glycyphagus, and Tyrophagus, of cockroaches, gnats, and fleas for example Blatella, Periplaneta, Chironomus, and Ctenocepphalides, mammalian allergens such as cat, dog, and horse, birds, venom allergens including those originating from insect stings or bites such as those of the taxonomic order Hymenoptera including bees (superfamily Apidae), wasps, and ants (superfamily Formicoidae). Still other allergenic antigens that may be used include inhalable allergens of fungi such as those of the genera Alternaria and Cladosporium.

Tumor antigens suitable as the first polypeptide of interest include, but are not limited to, MAGE, MART-1/Melan-A, gp100, dipeptidyl peptidase IV (DPPIV), adenosine deaminase binding protein (ADAbp), cyclophilin b, colorectal-associated antigen (CRC)-0017-1A/GA733, carcinoembryonic antigen (CEA) and its antigenic epitopes CAP-1 and CAP-2, etv6, aml1, prostate-specific antigen (PSA) and its antigenic epitopes PSA-1, PSA-2, and PSA-3, prostate-specific membrane antigen (PSMA), T cell receptor/CD3-ς chain, MAGE family of tumor antigens (for example, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE family of tumor antigens (for example, GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p2lras, RCAS1, α-fetoprotein, E-cadherin, β-catenin, 13-catenin, γ-catenin, pl2Octn, gp100Pme1117, PRAME, NY-ESO-1, cdc27, adenomatous polyposis colon protein (APC), fodrin, Connexin 37, Ig idiotype, p15, gp75, GM2 and GD2 gangliosides, viral products such as human papillomavirus proteins, Smad family of tumor antigens, Imp-1, P1A, EBV-encoded nuclear antigen (EBNA)-1, brain glycogen phosphorylase, SSX-1, SSX-2 (HOM-MEL-40), SSX-3, SSX-4, SSX-5, SCP-1, and CT-7, and c-erbB-2, acute lymphoblastic leukemia (etv6, amll, cyclophilin b), B-cell lymphoma (Ig idiotype), glioma (E-cadherin, a-catenin, 13-catenin, 7-catenin, p120ctn), bladder cancer (p2lras), bile duct cancer (p2lras), breast cancer (MUC family, HER2/neu, c-erbB-2), cervical carcinoma (p53, p2lras), colon carcinoma (p2lras, HER2/neu, c-erbB-2, MUC family), colorectal cancer (colorectal-associated antigen (CRC)-0017-1A/GA733, APC), choriocarcinoma (CEA), epithelial cell cancer (cyclophilin b), gastric cancer (HER2/neu, c-erbB-2, glycoprotein ga733), hepatocellular cancer, Hodgkin's lymphoma (Imp-1, EBNA-1), lung cancer (CEA, MAGE-3, NY-ESO-1), lymphoid cell-derived leukemia (cyclophilin b), melanoma (p15 protein, gp75, oncofetal antigen, GM2 and GD2 gangliosides, Melan-A/MART-1, cdc27, MAGE-3, p2lras, gp100Pme1117), myeloma (MUC family, p2lras), non-small cell lung carcinoma (HER2/neu, c-erbB-2), nasopharyngeal cancer (Imp-1, EBNA-1), ovarian cancer (MUC family, HER2/neu, c-erbB-2), prostate cancer (prostate-specific antigen (PSA) and its antigenic epitopes PSA-1, PSA-2, and PSA-3, PSMA, HER2/neu, c-erbB-2, glycoprotein ga733), kidney cancer (HER2/neu, c-erbB-2), squamous cell cancers of the cervix and esophagus (viral products such as human papillomavirus proteins), testicular cancer (NY-ES0-1), and T-cell leukemia (VLTH-1 epitopes).

The fusion protein of the invention has the capacity to form nanospheres and/or microspheres when expressed in a cell, regardless of whether it has a component of interest bound to the sortase recognition sequence or bound to the sortase acceptor motif. In that sense, the present invention also contemplates the formation of nanospheres and/or microspheres by means of the fusion protein of the invention, wherein said proteins do not comprise a component of interest bound to the sortase recognition sequence, and the subsequent modification of said nanospheres and/or microspheres through the addition of a component of interest to the sortase recognition sequence or to the sortase acceptor motif.

In that sense, in a particular embodiment of the nanosphere/microsphere of the invention, the fusion protein of the invention comprising component (ii)(a) further comprises a component of interest covalently bound to the residual part of the sortase recognition motif generated after a sortase-mediated reaction. In another particular embodiment of the nanosphere/microsphere of the invention, the fusion protein of the invention comprising component (ii)(b) further comprises a component of interest covalently bound to the sortase acceptor motif.

As is apparent to one skilled in the art, the modification of the nanosphere or microsphere by means of the sortase can produce an intermediate, wherein the fusion protein of the invention forming the nanosphere or microsphere is modified by means of cleavage of the sortase recognition motif. In that sense, in a particular embodiment, the nanosphere/microsphere of the invention is modified by means of the action of a sortase that recognizes the sortase recognition motif and cleaves said recognition motif. In another particular embodiment, the nanosphere/microsphere of the invention is modified by means of the action of a sortase that recognizes the sortase recognition motif and cleaves said sortase recognition motif, wherein the sortase recognition motif is amino acid sequence LPXTG (SEQ ID NO: 22), wherein X is any amino acid, and wherein the sortase cleaves between T and G of the sortase recognition motif.

Likewise, as is apparent to one skilled in the art, if the fusion protein of the invention comprises a sortase acceptor motif, the modification of the nanosphere or microsphere by means of the sortase simply involves the addition of a polypeptide to the amino end of the fusion protein without the cleavage of any polypeptide motif. In that sense, in a particular embodiment, the nanosphere/microsphere of the invention is modified by means of the action of a sortase that recognizes the sortase acceptor motif and covalently binds a polypeptide to that motif. In another particular embodiment, the nanosphere/microsphere of the invention is modified by means of the action of a sortase that recognizes the sortase acceptor motif, wherein the sortase recognition motif is the amino acid sequence selected from a group consisting of: G, A, GG, AA, GGG, and AAA, preferably GGG.

In another particular embodiment of the nanosphere/microsphere of the invention, the component of interest is selected from the group consisting of:
- toll-like receptor (TLR) ligands;
- monophosphoryl lipid A (MPL A);
- oligonucleotides selected from CpG islands and polyinosinic:polycytidylic acid (poly(1:C) - CAS number 24939-03-5);
- saponin;
- lipid molecules;
- coating molecules selected from a group consisting of: polyglycerols, polyethylene glycol, chitosans, poly(oxazolines) (POX), poly(hydroxypropyl methacrylate) (PHPMA), poly(2-hydroxyethyl methacrylate) (PHEMA), poly(N-(2-hydroxypropyl)methacrylamide) (HPMA), polyvinylpyrrolidone (PVP), poly(N,N-dimethylacrylamide) (PDMA), and poly(N-acryloylmorpholine) (PAcM);
- oligosaccharides;
- antibodies;
- nanoantibodies;
- affitins;
- viral antigen;
- bacterial antigen;
- fungal antigen;
- allergen;
- cell-penetrating peptides;
- affibody;
- environmental antigen; and
- a tumor antigen.

The term "toll-like receptor ligands" or "TLR" ligands, as used herein, refers to molecules having the capacity of binding to a protein of the family of toll-like receptor proteins. TLRs constitute a family of proteins that are part of the innate immune system. These receptors are transmembrane receptors and recognize molecular patterns expressed by a wide range of infectious agents and stimulate a variety of inflammatory responses.

The term "monophosphoryl lipid A" or the acronym "MPL A", as used herein, refers to a molecule derived from lipopolysaccharide (LPS), or endotoxin, which is highly immunostimulatory and has low toxicity, making it useful as a vaccine adjuvant.

The term "oligonucleotides", as used herein, refers to a single-stranded or double-stranded DNA or RNA sequence with 50 base pairs or less, wherein the bases are natural, i.e., adenine, cytosine, guanine, thymine, and uracil, or synthetic, and wherein the oligonucleotide may contain modifications, such as, without limitation, methylation (for example, 5-methylcytosine), phosphorylation, glycosylation, oxidation, etc.

The term "saponin", as used herein, refers to steroid or triterpenoid glycosides, so named because of their soap-like properties: each molecule consists of a lipid-soluble element (the steroid or triterpenoid) and a water-soluble element (the sugar), and they form a foam when shaken in water. Saponins are toxic, and their toxicity is believed to come from their ability to form complexes with sterols, so they could interfere with their assimilation by the digestive system or break down cell membranes after being absorbed into the bloodstream.

The term "lipid molecules", as used herein, refers to water-insoluble fatty substances including fats, oils, waxes, and related compounds.

The expression "coating molecules", as used herein, refers to any chemical component that can be used for coating the nanospheres/microspheres.

The term "oligosaccharides" in the present context refers to molecules formed by the covalent binding of 2 to 10 cyclic monosaccharides, where, when said molecules have 3 or more monosaccharides, they can be linear or branched by means of glycosidic bonds, a covalent bond established between the alcohol groups of two monosaccharides, with the release of a water molecule.

The term "antibody" (Ab) in the context of the present invention refers to an immunoglobulin molecule, a fragment of an immunoglobulin molecule, or a derivative of any of them, having the capacity to bind specifically to an antigen under typical physiological conditions. Immunoglobulin molecule heavy and light chain variable regions contain a binding domain which interacts with an antigen. The constant regions of antibodies (Abs) can mediate the binding of the immunoglobulin to host tissues or factors, including various cells of the immune system. It should also be understood that the term antibody, unless otherwise specified, also includes polyclonal antibodies, monoclonal antibodies (mAbs), antibody-like polypeptides, such as chimeric antibodies and humanized antibodies, and antibody fragments which retain the capacity to bind specifically to the antigen (antigen-binding fragments) provided by any known technique.

The term "nanoantibodies", also referred to as nanobodies, single-domain antibodies, or VHH antibodies, in the context of the present invention are a type of camelid antibodies which are much smaller than common antibodies, that are enormous by molecular standards, since each of them is an aggregate of two heavy chains and two light chains folded intricately and linked to complex sugars, while nanoantibodies are relatively simple proteins with an approximate size that is one-tenth the size of their human counterparts and only a few nanometers long.

The term "affitin", as used herein, refers to artificial proteins with the capacity to selectively bind to antigens.

The term "cell-penetrating peptides", or its acronym "CPPs", are short peptides that facilitate the cellular uptake/absorption of various molecular entities associated with the peptides, either by means of chemical bonds through covalent bonds or by means of non-covalent interactions. The associated molecular entities range from nanometric particles to small chemical molecules and large DNA fragments. The function of CPPs is to introduce the associated entities in cells, a process that usually occurs by endocytosis. CPPs normally have an amino acid composition that either contains a relatively high abundance of positively charged amino acids, such as lysine or arginine, or has sequences containing an alternating pattern of polar/charged amino acids and non-polar, hydrophobic amino acids. These two types of structures are referred to as polycationic or amphipathic, respectively. A third class of CPPs are hydrophobic peptides which contain only apolar residues with low a net charge or have hydrophobic amino acid groups that are crucial for cellular uptake.

In the context of the present description, the term "affibody" refers to a mimetic antibody that can bind to a specific target protein (receptor). Generally, an affibody molecule consists of 20 to 150 amino acid residues and can be made up of 2 to 10 alpha helices. Examples of affibodies are, without limitation, those described in Renli Luo et al. (RSC Chem. Biol., 2022, 3, 830-847).

The nanospheres/microspheres of the invention may have a variety of uses, such as, without any limitation, drug delivery vehicles, vehicles for the expression and purification of proteins, immunogenic agents, etc. This is possible due to the many modification and alteration possibilities presented by both the fusion protein and the nanospheres and microspheres.

In a particular embodiment, the component of interest further comprises a protease recognition sequence located in the C-terminal region of the sortase acceptor motif which characterizes the component of interest. Protease recognition sequences have been defined above and said definitions and particular embodiments are likewise valid for the present embodiment.

In a particular embodiment, the component of interest further comprises a signal peptide of the secretory pathway located in the C-terminal region of the sortase acceptor motif which characterizes the component of interest. Signal peptides of the secretory pathway have been defined above, and said definitions and particular embodiments are likewise valid for the present embodiment.

In a particular embodiment, the component of interest further comprises a tag to facilitate the purification thereof located in the C-terminal region of the sortase acceptor motif which characterizes the component of interest. Tags to facilitate purification have been defined above, and said definitions and particular embodiments are likewise valid for the present embodiment.

In another particular embodiment, the component of interest further comprises a sortase recognition sequence. Sortase recognition sequences have been defined above, and said definitions and particular embodiments are likewise valid for the present embodiment.

In a particular embodiment, the component of interest further comprises a tag to facilitate the purification thereof located in the sortase recognition sequence C-terminal region which characterizes the component of interest.

In a particular embodiment of the nanospheres and/or microspheres, the first polypeptide of interest, the component of interest, and/or the second polypeptide of interest is glucose-6-phosphatase 2 protein (IGRP) or a functionally equivalent variant.

The term "glucose-6-phosphatase 2" or "IGRP" or "islet-specific glucose-6-phosphatase catalytic subunit-related protein", as used herein, refers to a protein capable of hydrolyzing glucose-6-phosphate to yield glucose and phosphate. It is a transmembrane protein which is expressed in the pancreas and, to a lesser extent, in the testicles. In humans, it is encoded by the G6PC2 gene. The IGRP protein may be of any origin, for example human, bovine, murine, equine, canine, etc. In a particular embodiment, the IGRP protein is the human protein identified by accession number Q9NQR9 in the UniprotKB database (entry version 147, December 14, 2022, sequence version 1, October 1, 2000). In humans, there are three isoforms of the protein identified by the following UniprotKB accession numbers:
- Isoform 1, considered as the canonical sequence, with a length of 355 amino acids: Q9NQR9-1.
- Isoform 2, with a length of 102 amino acids: Q9NQR9-2.
- Isoform 3, with a length of 154 amino acids: Q9NQR9-3.

In another particular embodiment, the IGRP protein is the mouse protein identified by accession number Q9Z186 in the UniprotKB database (entry version 145, December 14, 2022; sequence version 1, May 1, 1999). In mouse, there are two isoforms of the protein identified by the following UniprotKB accession numbers:
- Isoform 1, considered as the canonical sequence, with a length of 355 amino acids: Q9Z186-1.
- Isoform 2, with a length of 154 amino acids: Q9Z186-2.

These isoforms as well as any other isoform of the IGRP protein of any origin are considered to be included in the term "IGRP" according to the present invention.

In another particular embodiment, the IGRP protein is the rat protein encoded by the gene identified by accession number Gene ID 681817 in the NCBI Genbank database (version of April 24, 2022).

The term "functionally equivalent variant", as used herein, referring to the IGRP protein, refers to any peptide or protein resulting from the deletion, insertion, addition, or substitution of one or more amino acid residues with respect to the sequence from which it is derived and which retains the function of said sequence.

In a particular embodiment, the functionally equivalent variant has a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% in the entire length thereof with the sequence of the IGRP protein.

As is apparent to one skilled in the art, there is no limitation whatsoever in the composition of the nanospheres/microspheres with respect to the fact that they may include one or more of the first polypeptide of interest, the component of interest, and the second polypeptide of interest.

Therefore, in a particular embodiment, the nanospheres and/or microspheres of the invention comprise the polypeptide of interest and/or the component of interest. In another particular embodiment, the polypeptide of interest is identical to or different from the component of interest.

Likewise, in a particular embodiment of the nanospheres and/or microspheres of the invention, the nanospheres/microspheres of the invention are bivalent or trivalent.

The term "nanospheres/microspheres bivalents", as used herein, refers to the nanospheres and/or microspheres comprising at least two different compounds/molecules/proteins from among the first polypeptide of interest, the component of interest, and the second polypeptide of interest. Similarly, the term "trivalent nanospheres/microspheres", as used herein, refers to the nanospheres and/or microspheres comprising at least three different compounds/molecules/proteins from among the first polypeptide of interest, the component of interest, and the second polypeptide of interest.

### Polynucleotides, vectors, and host cell of the invention

All the definitions and particular embodiments described above in relation to other aspects of the present invention are likewise applicable to the present aspects and their particular embodiments.

The fusion protein of the invention can be encoded by a polynucleotide. Therefore, another aspect of the present invention relates to a polynucleotide encoding a fusion protein of the invention, hereinafter the polynucleotide of the invention.

The term "polynucleotide", as used herein, refers to a polymer formed by a variable number of monomers wherein the monomers are nucleotides, including both ribonucleotides and deoxyribonucleotides. Polynucleotides include monomers modified by means of methylation, as well as non-modified forms. The terms "polynucleotide" and "nucleic acid" are used interchangeably in the present invention and include mRNA, cDNA, and recombinant polynucleotides.

Said polynucleotide can be part of an expression cassette, which is used, without limitation, for expressing said polynucleotide in a cell. Therefore, another aspect of the present invention relates to an expression cassette comprising a polynucleotide according to the invention, hereinafter the cassette of the invention.

The term "expression cassette", as used herein, refers to a polynucleotide comprising a gene and a promoter suitable for controlling said gene. The expression cassette may optionally include other sequences, for example, transcription termination signals. The choice of a promoter and other regulatory element(s) would generally vary based on the host cell used. Suitable promoters in the context of the present invention include constitutive promoters that promote the constant expression of the sequences associated therewith and inducible promoters that require an external stimulus to promote the transcription of the sequences associated therewith.

Promoters useful for the embodiment of the present invention include:
- Constitutive promoters such as, for example, the promoter of alcohol dehydrogenase (ADH1), the promoter of elongation factor-1-alpha (TEF), and the promoter of the gene encoding triose phosphate isomerase (TPI), the promoter of glyceraldehyde 3-phosphate dehydrogenase (GPD), and the promoter of 3-phosphoglycerate kinase (GPK), MRP7 promoter.
- Inducible promoters such as, for example, the promoter of metallothionein (CUP1) the expression of which is regulated by means of adding copper to the culture medium, the promoter of the gene encoding the FUS1 gene or the FUS2 gene the expression of which is activated in the presence of pheromones (factor α), the **TET** promoter the expression of which is regulated in the presence of tetracyclines, GAL1-10, GALL, GALS promoters which are activated in the presence of galactose, estrogen-inducible VP16-ER promoter, the promoter of phosphatase (PH05) the expression of which is activated in the presence of phosphate, and the promoter of heat shock protein HSP150 the expression of which is activated at a high temperature.

In a particular embodiment of the expression cassette of the invention, the cassette comprises a first polynucleotide and a second polynucleotide, wherein the second polynucleotide encodes a second fusion protein. In another particular embodiment, the polynucleotide of the invention or the expression cassette of the invention are operatively bound to a first promoter and the second polynucleotide is operatively bound to a second promoter. In another particular embodiment, the first promoter and the second promoter are the same promoter or are different promoters.

In a particular embodiment, when the cell in which the polynucleotide of the invention will be expressed is a bacterium, said promoter is the beta-lactamase and lactose promoter system, T7 RNA polymerase promoter, lambda promoter, trp promoter, or tac promoter. In a more particular embodiment of the invention, said promoter is an inducible promoter. In another more particular embodiment, said promoter is an inducible promoter that can be induced by isopropyl-β-D-1-thiogalactopyranoside (IPTG). In an even more particular embodiment, the inducible promoter that can be induced by IPTG is T7 RNA polymerase promoter.

In another aspect, the invention relates to a vector comprising the polynucleotide or the expression cassette of the invention, hereinafter the vector of the invention.

The term "vector", as used herein, refers to a nucleic acid sequence comprising the necessary sequences so that the fusion protein of the invention is generated after transcribing and translating said sequences in a cell. Said sequence is operatively bound to additional segments providing the autonomous replication thereof in a host cell of interest. Preferably, the vector is an expression vector, which is defined as a vector which, in addition to the regions for autonomous replication in a host cell, contains regions that are operatively linked to the polynucleotide of the invention and capable of enhancing the expression of the products of the polynucleotide according to the invention. The vectors of the invention can be obtained by means of techniques well known in the art. In a particular embodiment of the invention, the vector of the invention is a bacterial expression vector. In another particular embodiment of the invention, when the organism is a prokaryote, such as a bacterium, suitable vectors according to the invention are, for example, the vectors pUC18, pUC19, pUC118, pUC119, Bluescript and derivatives thereof, mp18, mp19, pBR322, pMB9, ColEl, pCRI, RP4, pNH8A, pNH16a, pNH18a. In a particular embodiment of the invention, when said bacterium is *E*. *coli,* said vector is plasmid pET, particularly plasmid pET Duet-1. Said plasmid comprises two different multiple cloning sites (MCS). Genes cloned into said plasmid are transcribed under the control of the T7 bacteriophage promoter, when T7 RNA polymerase is activated in the host cell. The expression is induced with IPTG (isopropyl-β-D-thiogalactopyranoside), which removes the repressor from the operator so as to carry out transcription and promote the expression of the protein of interest.

In a particular embodiment, the vector of the invention further comprises a second polynucleotide encoding a polypeptide selected from the group consisting of:
- a polypeptide comprising amino acids 477-542 (SEQ ID NO: 25) of the avian *Orthoreovirus* muNS protein,
- a polypeptide comprising amino acids 561-622 (SEQ ID NO: 26) of the mammalian *Orthoreovirus* muNS protein,
- a functionally equivalent variant of any of the above which retains the capacity to be incorporated into nanospheres and microspheres.

The different polynucleotides encoding the different components of the invention can be expressed from different vectors. Therefore, another aspect of the invention relates to a vector composition, hereinafter the vector composition of the invention, comprising the vector of the invention and a second vector comprising a second polynucleotide encoding a polypeptide selected from the group consisting of:
- a polypeptide comprising sequence 477-542 (SEQ ID NO: 25) of the avian *Orthoreovirus* muNS protein,
- a polypeptide comprising sequence 561-622 (SEQ ID NO: 26) of the mammalian *Orthoreovirus* muNS protein,
- a functionally equivalent variant of any of the above which retains the capacity to be incorporated into nanospheres.

Such as in the case of a single vector, the polynucleotides in the vector composition can also be operatively bound to promoters. In a particular embodiment of the vector composition of the invention, the polynucleotide of the invention or the expression cassette of the invention are operatively bound to a first promoter and the second polynucleotide is operatively bound to a second promoter. In another particular embodiment, the first promoter and the second promoter are the same promoter or are different promoters.

In a particular embodiment of the invention, the vectors of the vector composition of the invention are bacterial expression vectors. As will be apparent to one skilled in the art, the examples, definitions, and particular embodiments described for the vector of the invention are likewise valid for the vectors of the vector composition.

The polynucleotide of the invention, the expression cassette, the vector, or the vector composition of the invention may be comprised in a host cell. Another aspect of the present invention relates to a cell comprising a fusion protein of the invention, a polynucleotide of the invention, an expression cassette of the invention, a vector or vector composition of the invention, hereinafter the cell of the invention.

The cell of the invention can be any prokaryotic cell or any eukaryotic cell. In the present invention, virtually any cell type can be used. Any host cell which can be transformed with the polynucleotide of the invention, or which can be transformed, transfected, or infected with a recombinant vector containing the polynucleotide of the invention, for example, animal cells (such as mammalian cells, avian cells, insect cells, etc.), plant cells, yeasts, bacteria etc. The cells of the invention can be obtained by means of conventional methods known by those skilled in the art.

### Methods of the invention

All the definitions and particular embodiments described above in relation to other aspects of the present invention are likewise applicable to the present aspects and their particular embodiments.

Another aspect of the present invention relates to a method for producing a fusion protein of the invention, hereinafter method I of the invention, which comprises:
(a) expressing in a cell a first polynucleotide encoding said fusion protein,
(b) subjecting said cell to conditions suitable for the formation of nanospheres and/or microspheres, and
(c) concentrating the nanospheres and/or microspheres.

The first step of the method for producing the fusion protein of the invention comprises expressing in a cell a first polynucleotide encoding said fusion protein.

As one skilled in the art knows, in order to express the first polynucleotide in a cell, the polynucleotide must be introduced into the cell, for example, by means of transforming said cell with a vector comprising the polynucleotide. In the case where the polynucleotide is operatively bound to an inducible promoter, in order to express said polynucleotide it will be necessary to contact the cell with the inducer. In the particular case where the promoter is inducible by IPTG, in the case of bacterial cells, for the expression of the polynucleotide, IPTG is added to the bacterial culture for the time needed to achieve the expression of the polynucleotide, for example at least 30 minutes, at least 1 hour, at least 2 hours, at least 3 hours, at least 4 hours, at least 5 hours, at least 6 hours, at least 12 hours, at least 18 hours, at least 24 hours, at least 48 hours, at least 72 hours, or more. Preferably, IPTG induction is performed for a time period of about 3 hours. In a particular embodiment, cells are incubated during induction at a temperature of between 18 and 37°C, preferably between 25 and 37°C, more preferably 37°C. In a particular embodiment, when the induction time is more than 24 hours, cells are incubated during induction at a temperature of between 18 and 25°C. Once induction has ended, bacteria are collected by centrifugation.

The second step of the method for producing the fusion protein of the invention consists of subjecting the cells to conditions suitable for the formation of nanospheres and/or microspheres.

Conditions suitable for the formation of nanospheres and/or microspheres can be determined by one skilled in the art for each cell type. Methods suitable for detecting the formation of nanospheres and/or microspheres include, but are not limited to, the method described in Example 1 of patent application WO 2011/098652, wherein the formation of nanospheres/microspheres (inclusions) is detected by means of indirect immunofluorescence microscopy using anti-muNS polyclonal antibodies. In a particular embodiment, the conditions suitable for the formation of nanospheres and/or microspheres comprise incubating the cells expressing the first polynucleotide for a time period of at least 30 minutes, at least 1 hour, at least 2 hours, at least 3 hours, at least 4 hours, at least 5 hours, at least 6 hours, at least 12 hours, at least 18 hours, at least 24 hours, and at a temperature of between 25 and 37°C, preferably 37°C.

In a particular embodiment of method I of the invention, the cell is a bacterial cell or a eukaryotic cell, wherein if it is a bacterial cell, nanospheres are formed in step (b), and if it is a eukaryotic cell, microspheres are formed in step (b).

The first step and the second step of the method for producing the fusion protein of the invention can be carried out simultaneously or sequentially, such that first the expression of the first polynucleotide is carried out and then the cells are subjected to conditions suitable for the formation of nanospheres and/or microspheres. In a particular embodiment, the first and second steps are carried out simultaneously, given that the conditions to which the cells are subjected for the expression of the first and second polynucleotides are suitable for the formation of microspheres. In a more particular embodiment, said conditions comprise incubating the cells in the presence of the inducer, preferably IPTG, for a time period of at least 30 minutes, at least 1 hour, at least 2 hours, at least 3 hours, at least 4 hours, at least 5 hours, at least 6 hours, at least 12 hours, at least 18 hours, at least 24 hours, and at a temperature of between 25 and 37°C, preferably 37°C.

The third step of method I consists of concentrating the nanospheres and/or microspheres. In order to concentrate the nanospheres/microspheres, the cells in which nanospheres/microspheres have been formed must be lysed by means of any method, such as incubation with a lysis buffer or sonication, among others. Then, deposition of the nanospheres/microspheres can be performed by means of centrifugation, preferably at a speed of about 2700 g. In a particular embodiment, once the cells have been lysed, the nanospheres/microspheres are washed with a buffer comprising a divalent cation. The term "divalent cation", as used herein, refers to a positively charged ion of any metal from the periodic table having a valence of 2. Divalent cations suitable for use thereof in the present invention include, without limitation, the divalent cations of Mg, Cd, Ca, Co, Cu, Fe, Mn, Ni, Sr, and Zn. In a preferred embodiment, the divalent cation is Mg²⁺. Suitable divalent cation concentrations to induce the formation of muNS protein aggregates are, for example, at least 0.5 mM, at least 0.8 mM, at least 1 mM, at least 5 mM, at least 10 mM, at least 15 mM, at least 20 mM, or above. In a particular embodiment, the nanospheres and/or microspheres are washed with a buffer comprising Mg²⁺, for example MgCl₂, preferably at a concentration of 5 mM. In an even more particular embodiment, the nanospheres and/or microspheres are concentrated following the purification protocol indicated in the examples of the present document.

In a particular embodiment, method I of the invention further comprises purifying the fusion protein by separating it from the nanospheres and/or microspheres.

In the case where the fusion protein of the invention comprises component (ii)(b) and a protease recognition sequence fused to the amino end of the sortase acceptor motif, it may be useful to cleave the protease recognition sequence such that the sortase acceptor motif is available to interact with the sortase. Therefore, in a particular embodiment of method I of the invention, if the fusion protein comprises component (ii)(b) and a protease recognition sequence fused to the amino end of the sortase acceptor motif, the method further comprises contacting the fusion protein with a protease specific for the protease recognition sequence that is fused to the amino end of the sortase acceptor sequence, under conditions suitable for the proteolysis of said recognition sequence, with the subsequent separation of the protease recognition sequence of the fusion protein. The conditions suitable for the proteolysis of said protease recognition sequence are described in this description and exemplified in the examples described herein.

In order to separate the fusion protein from the nanospheres and/or microspheres, they must be subjected to conditions leading to the disintegration thereof. Therefore, in a particular embodiment, method I of the invention further comprises purifying the fusion protein by separating it from the nanospheres and/or microspheres, subjecting to that end the nanospheres and/or microspheres to conditions leading to the disintegration thereof.

The conditions leading to the disintegration of the nanospheres and/or microspheres include, among others:
- incubation of the nanospheres and/or microspheres with denaturing agents, for example, urea or guanidinium hydrochloride,
- incubation of the nanospheres and/or microspheres with ionic detergents, for example, high-concentration SDS,
- incubation with NaCl at concentrations above 500 mM,
- incubation with a buffer which does not comprise divalent cations, preferably which does not comprise Mg²⁺.

In a particular embodiment, the conditions leading to the disintegration of the nanospheres and/or microspheres are incubation with a buffer which does not comprise divalent cations, preferably which does not comprise Mg²⁺.

In a particular embodiment, once the nanospheres and/or microspheres have disintegrated, the fusion protein can be purified by means of any known method. Said method will depend on the nature of the polypeptide of interest and will be known by one skilled in the art. Polypeptide purification techniques are well known in the art and include, without limitation, affinity chromatography, exclusion chromatography, ion exchange chromatography, adsorption chromatography, immunoprecipitation, etc.

In addition to being able to be modified by a sortase and incorporate a component of interest into the fusion protein of the invention, the nanosphere/microsphere of the invention can be used as a platform for the incorporation of a second fusion protein comprising the IC region of the *Orthoreovirus* muNS protein. This permits the nanosphere/microsphere of the invention to be used to produce proteins using said second fusion protein.

Therefore, another aspect of the present invention relates to a method for producing a protein, hereinafter method II of the invention, which comprises:
(a) expressing in a cell a first polynucleotide encoding a fusion protein of the invention and a second polynucleotide encoding a second fusion protein comprising components:
   (i) a polypeptide selected from the group consisting of:
      - a polypeptide comprising sequence 477-542 (SEQ ID NO: 25) of the avian *Orthoreovirus* muNS protein,
      - a polypeptide comprising sequence 561-622 (SEQ ID NO: 26) of the mammalian *Orthoreovirus* muNS protein,
      - a functionally equivalent variant of any of the above which retains the capacity to be incorporated into nanospheres and/or microspheres, and
   (ii) a second polypeptide of interest,
   wherein component (ii) is the protein that is produced;
(b) subjecting said cell to conditions suitable for the formation of nanospheres or microspheres, and
(c) concentrating the nanospheres or microspheres.

In a particular embodiment, method II of the invention comprises purifying the fusion protein by separating it from the nanospheres or microspheres.

In a particular embodiment of method II of the invention, component (ii) is fused to the amino end of component (i) or to the carboxyl end of component (i).

As one skilled in the art will understand, the separation of component (ii) from component (i) in method II of the invention may be useful. One possibility to enable separating said components is the use of a peptide the sequence of which contains a cleavage target for a protease, thereby permitting the separation of the two components.

In a particular embodiment of method II of the invention, the second fusion protein comprises a protease recognition sequence between its components (i) and (ii).

The term "protease" or "peptidases", as used herein, refers to enzymes that break the peptide bonds of proteins, using for that purpose a water molecule, so they are classified as hydrolases. Examples of protease cleavage sites suitable for incorporation into the fusion protein of the invention include, without limitation, enterokinase (cleavage site DDDDK; SEQ ID NO: 5), factor Xa (cleavage site IEDGR; SEQ ID NO: 6), thrombin (cleavage site LVPRGS; SEQ ID NO: 7), protease TEV (cleavage site ENLYFQG; SEQ ID NO: 8), protease PreScission (cleavage site LEVLFQGP; SEQ ID NO: 9), inteins, and the like. In a particular embodiment, the protease recognition sequence is an enterokinase recognition sequence or a factor Xa recognition sequence.

In a particular embodiment of method II of the invention, the second fusion protein comprises a protease recognition sequence between its components (i) and (ii), and method II of the invention further comprises a step of separating the protein that is produced from component (i) of the second fusion protein through incubation of the second protein with a protease in conditions leading to the cleavage of the recognition sequence by the protease.

In another particular embodiment, method II of the invention further comprises purifying the fusion protein by separating it from the nanospheres and/or microspheres by subjecting the nanospheres and/or microspheres to conditions leading to the disintegration thereof.

In another particular embodiment of method II of the invention, the cell is a bacterial cell or a eukaryotic cell, wherein if it is a bacterial cell, nanospheres are formed in step (b), and if it is a eukaryotic cell, microspheres are formed in step (b).

In another particular embodiment of method II of the invention, the protein is the IGRP protein.

Another aspect of the invention relates to a method for producing a first polypeptide of interest, hereinafter method III of the invention, wherein the method comprises the following steps:
(a) producing the fusion protein of the invention by means of method I of the invention, wherein the fusion protein comprises:
   - component (ii)(a) and a component (iii) fused to the amino end of component (i), wherein component (iii) is the polypeptide of interest, and
   - a protease recognition sequence between its components (i) and (iii),
(b) subjecting the nanospheres and/or microspheres to conditions leading to the disintegration thereof, causing the separation of the fusion protein and the nanospheres or microspheres,
(c) contacting the product resulting from step (b) with a protease specific for the recognition sequence connecting components (i) and (iii) of the fusion protein under conditions suitable for the proteolysis of said fusion protein, with the subsequent separation of components (i) and (iii) of the fusion protein,
(d) subjecting the product of step (c) to conditions suitable for the formation of the nanospheres or microspheres, and
(e) separating the nanospheres or microspheres from component (iii).

In a particular embodiment, the protease recognition sequence binding components (i) and (iii) of the fusion protein is an enterokinase recognition sequence (cleavage site DDDDK- SEQ ID NO: 5), factor Xa recognition sequence (cleavage site IEDGR- SEQ ID NO: 6), thrombin recognition sequence (cleavage site LVPRGS- SEQ ID NO: 7), protease TEV recognition sequence (cleavage site ENLYFQG - SEQ ID NO: 8), protease PreScission recognition sequence (cleavage site LEVLFQGP - SEQ ID NO: 9), intein recognition sequence, or the like. In a more particular embodiment, the protease recognition sequence is an enterokinase recognition sequence or a factor Xa recognition sequence. In an even more particular embodiment, the protease recognition sequence is the sequence of SEQ ID NO: 5 or the sequence of SEQ ID NO: 6.

Conditions suitable for the proteolysis of the fusion protein comprise contacting the fusion protein with the protease specific for the recognition sequence connecting components (i) and (iii) of the fusion protein under pH conditions, temperature conditions, etc., that will depend on the specific protease to be used. One skilled in the art knows how to determine these conditions for each particular protease.

Conditions suitable for the formation of nanospheres and/or microspheres include contacting the product resulting from step (a) with a buffer containing a divalent cation, as described above. In a preferred embodiment, the divalent cation is Mg²⁺. Suitable divalent cation concentrations to induce the formation of muNS protein aggregates are, for example, at least 0.01 mM, at least 0.1 mM, at least 1 mM, at least 2 mM, at least 3 mM, at least 4 mM, at least 5 mM, or above. In a particular embodiment, said conditions comprise incubation with a buffer comprising Mg²⁺, preferably at a concentration of 5 mM.

According to the present method, when the nanospheres and/or microspheres form component (i) of the fusion protein again, it is again integrated in said nanospheres and/or microspheres, with component (iii) of the fusion protein being free. Therefore, this is a method suitable for protein purification.

Another aspect of the present invention relates to a protein obtainable according to method I, II, or III of the invention.

Another aspect of the present invention relates to a method for producing a nanosphere or microsphere of the invention, hereinafter method IV of the invention, comprising the steps of:
(a) producing the fusion protein by means of method I of the invention, and
(b) subjecting said cell to conditions suitable for the formation of nanospheres or microspheres.

In a particular embodiment of method IV of the invention, the cell is a bacterial cell or a eukaryotic cell, wherein if it is a bacterial cell, nanospheres are formed in step (b), and if it is a eukaryotic cell, microspheres are formed in step (b).

Conditions for incubating the nanospheres or microspheres in the presence of a sortase are known by one skilled in the art.

In a particular embodiment, method IV of the invention further comprises the step of incubating the nanospheres and/or microspheres in the presence of a sortase that recognizes the sortase recognition motif of component (ii)(a) of the fusion protein and a substrate comprising a sortase acceptor motif, obtaining a nanosphere and/or microsphere comprising the substrate that comprises the sortase acceptor motif.

In a particular embodiment, method IV of the invention further comprises the step of incubating the nanospheres and/or microspheres in the presence of a sortase that recognizes the sortase acceptor motif in component (ii)(b) of the fusion protein and a substrate comprising a sortase recognition motif, obtaining nanospheres and/or microspheres comprising the substrate that comprises the recognition motif of sortase.

In a particular embodiment of method IV of the invention, the sortase is sortase A, the recognition motif is the sequence SEQ ID NO: 22 or SEQ ID NO: 23, and the sortase acceptor motif is a polyglycine sequence, preferably sequence GGG.

In a particular embodiment of method IV of the invention, the step of incubating the nanospheres and/or microspheres in the presence of a sortase is performed in the presence of an excess of calcium ions (Ca²⁺) and in the absence of phosphate groups (PO₄³⁻). In another particular embodiment of method IV of the invention, the step of incubating the nanospheres and/or microspheres in the presence of a sortase is performed at a pH of between 6.5 to 8.5, preferably at a pH of 7.5, at a temperature of between 35°C and 38°C, preferably 37°C, for 3 hours (h) to 5 h, preferably 4 h.

In another particular embodiment of method IV of the invention, the step of incubating the nanospheres and/or microspheres in the presence of a sortase is performed in the presence of an excess of calcium ions and the absence of phosphate groups at a pH of 7.5 for 4 h.

In another particular embodiment of method IV of the invention, the reaction during the step of incubating the nanospheres and/or microspheres in the presence of a sortase is stopped with the addition of an excess of ethylenediaminetetraacetic acid (EDTA) (CAS number: 60-00-4).

In another particular embodiment of method IV of the invention, the nanospheres or microspheres are purified between step (b) and the step of incubating the nanospheres and/or microspheres in the presence of a sortase.

### Pharmaceutical compositions

All the definitions and particular embodiments described above in relation to other aspects of the present invention are likewise applicable to the present aspects and their particular embodiments.

The fusion protein of the invention and the nanosphere or microsphere of the invention can be used to develop pharmaceutical compositions and in therapeutic uses.

Therefore, another aspect of the present invention relates to a pharmaceutical composition or immunogenic composition, hereinafter the pharmaceutical composition of the invention or the immunogenic composition of the invention, comprising the nanosphere and/or microsphere of the invention and a pharmaceutically acceptable excipient.

The term "immunogenic composition" refers to a composition which is capable of causing, establishing, or inducing an immune response, i.e., a cellular immune response or an antibody-mediated immune response, to the composition after administration in a subject. An "immunogenic composition" comprises molecules with antigenic properties, such as dead or attenuated bacteria or viruses, and also immunogenic polypeptides. An immunogenic polypeptide is generally referred to as antigenic. A molecule is "antigenic" when it is capable of interacting specifically with an antigen recognition molecule of the immune system such as an immunoglobulin (antibody) or a T-cell antigen receptor. It should be understood that, in the present invention, the immunogenic composition of the invention comprises the fusion protein of the invention and optionally the nanospheres or microspheres of the invention.

As will be apparent to one skilled in the art, a vaccine is an immunogenic composition. Therefore, in a particular embodiment, the immunogenic composition is a vaccine or vaccine composition. The term "vaccine" or "vaccine composition", as used herein, refers to an immunogenic composition which is capable of causing, establishing, inducing, or improving an immune response against a particular disease, wherein said immune response is a cell-type immune response or an antibody-mediated immune response, after prophylactic administration to the subject. A vaccine or vaccine composition normally contains an agent which is similar to a microorganism causing the disease or to a part thereof (for example, a polypeptide). The vaccines or vaccine compositions can be prophylactic or therapeutic. The term "vaccine composition", as used herein, refers to an immunogenic composition of the invention complemented with pharmaceutically acceptable carrier excipients which, when administered to a subject, causes or is capable of causing, directly or indirectly, a protective immune response against the microorganism causing the disease in the host or subject.

"Pharmaceutically acceptable excipient" is understood to mean a therapeutically inactive substance which is used to incorporate the active ingredient and is acceptable for the patient from a pharmacological/toxicological viewpoint and for the pharmaceutical chemist who prepares it from a physical/chemical viewpoint with respect to composition, formulation, stability, patient acceptance, and bioavailability. The excipient or vehicle also includes any substance that serves to improve the administration and effectiveness of the active ingredient within the pharmaceutical composition. Examples of pharmaceutically acceptable vehicles include one or more of water, saline solution, phosphate buffered saline solution, dextrose, glycerol, ethanol, and the like, as well as combinations thereof. In many cases, it will be preferable to include isotonic agents, for example, sugars, sugar alcohols such as mannitol, sorbitol, or sodium chloride in the composition. Pharmaceutically acceptable vehicles may further comprise small amounts of auxiliary substances such as wetting or emulsifying agents, preservatives, or buffers, which increase the shelf life or efficacy of the microspheres or the compositions that are part of the pharmaceutical compositions. Examples of suitable vehicles are well known in the literature (see, for example, Remington's Pharmaceutical Sciences, 19th ed., Mack Publishing Company, Easton, PA, 1995). In some cases, a disintegrating agent such as cross-linked polyvinylpyrrolidone, agar, alginic acid, or sodium alginate, can be added. The number and the nature of the pharmaceutically acceptable excipients depend on the desired dosage form. The pharmaceutically acceptable excipients are known by those skilled in the art (Faulí and Trillo C. (1993) "Tratado de Farmacia Galénica", Luzán 5, S.A. Editions, Madrid).

The pharmaceutical compositions of the invention can be administered by any suitable route, such as oral, subcutaneous, intravenous, intraperitoneal, or intramuscular.

### Medical uses

All the definitions and particular embodiments described above in relation to other aspects of the present invention are likewise applicable to the present aspects and their particular embodiments.

As will be apparent to one skilled in the art, the fusion protein and/or the nanospheres or microspheres find use in medicine. Therefore, another aspect of the invention relates to the nanospheres and/or microspheres of the invention for use in medicine.

Another aspect of the present invention relates to the nanospheres and/or microspheres of the invention or the immunogenic composition of the invention for use in the treatment and/or prevention of bluetongue, hereinafter the first medical use of the invention, wherein the fusion proteins comprised in the nanosphere/microsphere are characterized by comprising at least one of:
- the bluetongue virus 4 (BTV) outer capsid protein 2 (VP2);
- the bluetongue virus 10 (BTV) core protein 7 (VP7);
- the bluetongue virus 4 (BTV) non-structural protein 1 (NS1);
- a protein with a sequence functionally equivalent to any of the above.

The term "bluetongue" or "catarrhal fever of sheep" is a non-contagious viral disease which affects domestic and wild ruminants (mainly sheep, but also bovines, goats, buffalo, antelopes, deer, or moose) and is transmitted by mosquitos of the Culicoides species. The virus that causes bluetongue, bluetongue virus (BTV), is identified as a member of the *Orbivirus* genus of the family Reoviridae. Bluetongue virus species, or serogroup, encompasses 24 known serotypes and other atypical serotypes that are recently described. In many animals, bluetongue virus (BTV) infection may not be noticeable, but it may cause a deadly disease in some infected ruminants. Disease severity varies between different species and strains, with the most serious symptoms being observed in sheep, causing death, weight loss, and impaired wool growth. In highly susceptible sheep, morbidity may reach 100%, while mortality ranges from 2 to 30%, but may reach 70%.

The term "outer capsid protein 2" or "VP2", as used herein, refers to one of the two proteins (with VP5) which constitute the outer capsid of viral BTV particle. VP2 is the main immunogenic particle of BTV. VP2 is responsible for the adhesion of the virus to the target host cell, probably as a result of binding to sialic acid. This binding induces the internalization of the virion predominantly by clathrin-dependent endocytosis. In a particular embodiment of the treatment of bluetongue of the invention, BTV4 outer capsid protein 2 comprises the sequence identified by accession number P12434 in the UniprotKB database (entry version 70, December 14, 2022; sequence version 2, November 1, 1991).

The term "core protein 7" or "VP7", as used herein, refers to one of BTV core proteins, which is accessible from the surface of an intact virus. VP7 seems to be important for the interactions of the virus with insect cells. In a particular embodiment of the treatment of bluetongue of the invention, BTV4 core protein 7 comprises the sequence identified by accession number P69361 in the UniprotKB database (entry version 77, December 14, 2022; sequence version 1, March 3, 2005).

The term "non-structural protein 1" or "NS1", as used herein, refers to a positive viral protein synthesis regulator. In a particular embodiment of the treatment of bluetongue of the invention, BTV4 core protein 7 comprises the sequence identified by accession number Q1W9P8 in the UniprotKB database (entry version 16, December 14, 2022; sequence version 1, May 2, 2006).

**In** the case of the first medical use of the invention, the expression "functionally equivalent sequence" refers to VP2, VP7, or NS1 protein variants which completely or partially retain their capacity to function as immunogenic polypeptides, as described above.

Functionally equivalent VP2, VP7, or NS1 protein variants include those showing at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

**In** the context of the invention, "treatment and/or prevention of bluetongue" is understood to mean the administration of the nanospheres and/or microspheres of the invention to prevent or delay the onset of symptoms, complications, or biochemical indications of the infection, to alleviate its symptoms, or to stop or inhibit its development and progression such as, for example, death. Treatment can be a prophylactic treatment to prevent the onset of the disease or to prevent the manifestation of its clinical or subclinical symptoms or a therapeutic treatment to eliminate or alleviate symptoms after the manifestation of the disease.

**In** a particular embodiment of the first medical use of the invention, treatment is performed in a subject.

The term "patient" or "subject", as used herein, refers to any animal, preferably a mammal and includes, among others, domestic and farm animals, primates, and human beings, for example, human beings, non-human primates, cows, horses, pigs, sheep, goats, dogs, cats, or rodents like rats and mice. **In** a preferred embodiment, the subject is a human being of any age or race.

**In** a particular embodiment, the first medical use of the invention comprises the administration of a therapeutically effective amount of the nanospheres and/or microspheres.

**In** another particular embodiment of the first medical use of the invention, the nanosphere and/or microsphere is administered in the absence of an adjuvant. Without wishing to be bound to a particular theory, it is considered that the administration of antigens in the absence of adjuvant favors the development of a tolerance response instead of an inflammatory response in the body.

The expression "therapeutically effective amount", as used herein, is understood to mean an amount which is capable of providing a therapeutic effect and can be determined by one skilled in the art through commonly used means. In particular, the therapeutically effective amount of the nanospheres and/or microspheres of the invention is the amount capable of causing an immune response in the subject. The amount of nanospheres and/or microspheres of the invention which can be included in the pharmaceutical compositions according to the invention will vary depending on the subject and the particular mode of administration. Those skilled in the art will appreciate that dosages can also be determined under the guidance of Goodman and Goldman's The Pharmacological Basis of Therapeutics, ninth edition (1996), appendix II, pages 1707-1711 and Goodman and Goldman's The Pharmacological Basis of Therapeutics, twelfth edition (2001), appendix II, pages 475-493.

Another aspect of the present invention relates to the nanospheres and/or microspheres of the invention or the immunogenic composition of the invention for use in the treatment and/or prevention of African horse sickness, hereinafter the second medical use of the invention, wherein the fusion proteins comprised in the nanosphere are characterized by comprising at least one of:
- the African horse sickness virus (AHSV) non-structural protein 1 (NS1);
- a protein with a sequence functionally equivalent to any of the above.

The term "African horse sickness virus" or "AHSV", as used herein, describes the *Orbivirus* AHSV of the family Reoviridae with nude, 60-80 nm, icosahedral virion which is highly similar to agents responsible for bluetongue in sheep or epizootic hemorrhagic disease in deer. AHSV is resistant to organic solvents and bile salts, but sensitive to non-neutral pHs, heat, and putrefaction. It has 9 serotypes with common complement fixation antigens, but with crossed protection only between 6 and 9. **It** has variable virulence, which is the highest in serotype 4, with mortality above 90%, and the lowest in serotype 9, ranging between 70 and 80%. AHSV causes an acute viral horse disease which is transmitted by arthropods, has high mortality, and occurs seasonally with acute fever, cardiac and pulmonary cases, and edematous and hemorrhagic lesions.

**In** a particular embodiment of the second medical use of the invention, AHSV non-structural protein 1 comprises the sequence identified by accession number P87505 in the UniprotKB database (entry version 49, December 14, 2022; sequence version 1, May 1, 1997).

The causal agent is *Orbivirus* AHSV of the family Reoviridae with nude, 60-80 nm, icosahedral virion which is highly similar to agents responsible for bluetongue in sheep or epizootic hemorrhagic disease in deer. AHSV is resistant to organic solvents and bile salts, but sensitive to non-neutral pHs, heat, and putrefaction. It can be cultured in HeLa and BHK 21 cells and isolated in an embryonated egg and a suckling mouse. It has 9 serotypes with common complement fixation antigens, but with crossed protection only between 6 and 9. It has variable virulence, which is the highest in serotype 4, with mortality above 90%, and the lowest in serotype 9, ranging between 70 and 80%.

**In** the case of the second medical use of the invention, the expression "functionally equivalent sequence" refers to AHSV NS1 protein variants which completely or partially retain their capacity to function as immunogenic polypeptides, as described above.

Functionally equivalent NS1 protein variants include those showing at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

**In** a particular embodiment of the second medical use of the invention, treatment is performed in a subject, preferably a mammal and includes, among others, domestic and farm animals, primates, and human beings, for example, human beings, non-human primates, cows, horses, pigs, sheep, goats, dogs, cats, or rodents like rats and mice.

**In** a particular embodiment, the second medical use of the invention comprises the administration of a therapeutically effective amount of the nanospheres and/or microspheres. **In** another particular embodiment of the treatment of African horse sickness of the invention, the nanosphere is administered in the absence of an adjuvant.

Another aspect of the present invention relates to the nanospheres and/or microspheres of the invention or the pharmaceutical composition of the invention for use in the treatment and/or prevention of type 1 diabetes, hereinafter the third medical use of the invention, wherein the fusion protein comprised in the nanosphere and/or microsphere is characterized by comprising the glucose-6-phosphatase 2 protein (IGRP) according to the sequence with accession number Q9NQR9 in the UniProt database, entry dated August 3, 2022, sequence version 1.

The term "type 1 diabetes", or "diabetes mellitus type I", or "juvenile diabetes", or "insulin-dependent diabetes mellitus", as used herein, refers to a metabolic disease characterized by a selective destruction of beta cells in the pancreas, causing complete insulin deficiency. It differs from type 2 diabetes because it is a type of diabetes characterized by its early occurrence in life, generally before 30 years of age. Only 1 of every 20 people with diabetes has type 1 diabetes which occurs more frequently in young people and children. Type 1 diabetes is classified into autoimmune cases, i.e., the most common form, and idiopathic cases. The term "type 1 diabetes", as used herein, includes both classic type 1 diabetes, generally diagnosed before 30 years of age and requires insulin treatment from the moment of diagnosis, and latent autoimmune diabetes in adults, diagnosed after 30 years of age and does not usually require insulin treatment in the 3-6 months following diagnosis.

In a particular embodiment of the third medical use of the invention, the type 1 diabetes is autoimmune type 1 diabetes. The term "autoimmune type 1 diabetes", as used herein, refers to an autoimmune disease involving a selective destruction of β cells in the pancreas mediated by T lymphocytes activated in subjects with predisposing HLA haplotypes. After a preclinical period of variable duration, during which the patient remains asymptomatic, when the insulin-producing cell mass reaches a critical value, the patient exhibits classic symptomatology: polyuria, polydipsia, polyphagia, weight loss, and progressive ketosis which may lead to in ketoacidosis, if no exogenous insulin treatment is introduced.

In a more particular embodiment of the third medical use of the invention, the type 1 diabetes is "latent autoimmune diabetes in adults" or "LADA", and as used herein, it refers to a type of autoimmune diabetes diagnosed in adulthood, normally after 30 years of age, in subjects who are positive for at least one autoantibody from those which are normally present in patients with classic type 1 diabetes, for example, islet cell antibodies (ICA), glutamic acid decarboxylase antibodies (GADA), islet antigen-2 antibodies (IA-A2), or insulin autoantibodies (IAA), and who do not require insulin treatment in the first 3 or 6 months following diagnosis. Autoimmune diabetes in adults differs from type 1 diabetes due to slowly progressing β cell failure and the subsequent gradual dependence on insulin. Normally, in patients with LADA, the function of β cells is affected within six years of diagnosis.

In a preferred embodiment of the third medical use of the invention, the subject is a human being of any age or race. In a particular embodiment, the subject is at risk of developing type 1 diabetes. A subject at risk of developing type 1 diabetes is an individual who has a direct family member (brother/sister) with diagnosed type 1 diabetes and who furthermore has multiple (≥2) serum autoantibodies targeting pancreatic islets (ICA), glutamic acid decarboxylase 65 (GADA65), islet antigen-2 (IA-2), or ZnT8 transporter (ZnT8).

A particular embodiment of the third medical use of the invention comprises the administration of a therapeutically effective amount of the nanospheres and/or microspheres. In another particular embodiment of the treatment of type 1 diabetes of the invention, the nanosphere and/or microsphere is administered in the absence of an adjuvant.

In another particular embodiment of the third medical use of the invention, the administration of the nanosphere and/or microsphere is by subcutaneous or intravenous route.

Another aspect of the present invention relates to a method for inducing type 1 diabetes in an animal model, hereinafter method V of the invention, which comprises administering to an animal an effective amount of the nanosphere and/or microsphere of the invention, wherein the fusion protein comprised in the nanosphere and/or microsphere is characterized by comprising the glucose-6-phosphatase 2 protein (IGRP).

In a particular embodiment, the glucose-6-phosphatase 2 protein (IGRP) has the sequence with accession number Q9NQR9 in the UniProt database, entry dated August 3, 2022, sequence version 1.

The IGRP protein is capable of inducing diabetes when administered to a mouse in the form of a DNA vaccine (Fuchs et al., Clinical and Experimental Immunology, 2014, 176: 199-206). In a particular embodiment, the functionally equivalent variants of the IGRP protein retain the capacity to induce diabetes of the IGRP protein from which they are derived. The capacity of a protein to induce diabetes in an animal model can be determined by means of methods known by one skilled in the art, for example, by means of determining urine or blood glucose levels. For example, Fuchs *et al. (supra)* considered that the murine model presents diabetes when two consecutive measurements of urine glucose levels above 5.5 mmol/L or blood glucose levels above 13.9 mmol/L are obtained. Furthermore, the IGRP protein exhibits glucose 6-phosphatase activity, i.e., it is capable of catalyzing the dephosphorylation of glucose-6-phosphate to glucose. In a particular embodiment of method V of the invention, the functionally equivalent variants of the IGRP protein retain the phosphatase activity of the IGRP protein from which they are derived. Glucose-6-phosphatase activity can be determined by means of methods known by one skilled in the art, for example, methods based on the detection of inorganic phosphate in aqueous solutions, such as the malachite green method (Petrolonis *et al., supra),* or methods based on the detection of glucose-6-phosphate such as, for example, the methods based on the glucose oxidase/peroxidase system (Mao, H., et al., Anal. Chem. 2002, 74, 379-385).

In a particular embodiment of method V of the invention, the method comprises administering to an animal an effective amount of the nanosphere and/or microsphere of the invention.

The term "effective amount" refers to the amount of nanospheres and/or microspheres that leads to the subject, to whom the nanosphere and/or microsphere is administered, being considered as having diabetes.

In another particular embodiment of method V of the invention, the administration is by subcutaneous or intramuscular route.

In another particular embodiment of method V of the invention, the nanosphere and/or microsphere is administered together with an adjuvant.

The following examples serve to illustrate the invention and should not be considered as limiting the scope thereof.

### EXAMPLES

### Example 1

### Materials and Methods and Results

### muNS-HA or HA-muNS does not form cytoplasmic inclusions

A protein labeled with hemagglutinin epitope (HA) fused at the carboxyl terminal end of the protein was created to purify the muNS-Mi protein. Analysis of its expression in HeLa cells by means of immunofluorescence demonstrated that tag addition at the C-terminal end of the muNS-Mi protein led to the loss in muNS-Mi's capacity to form cytoplasmic inclusions (Figure 4).

### Construction of plasmids pET Duet 1- MiST and pET Duet 1-MiST/IC-AvPAL

The muNS-Mi sequence was obtained by means of PCR amplification from plasmid pCIneo-muNS (488-635) (Brandariz-Nuñez et al. A 2010, PLoS ONE 5(11) e13785) using as a positive primer 5'-CATGCCATGGCACCAGCCGTACTGCTGTC-3' (target Ncol underlined and ATG initiator double underlined) (SEQ ID NO: 27) and as a negative primer 5'-TTGCGGCCGCAATCAGCCGGTTTCCGGCAGCAGATCATCCACC -3' (target Notl underlined and stop codon double underlined) (SEQ ID NO: 28) which contains the sortase motif sequence followed by a codon stop. The amplified product was inserted into the first polylinker of plasmid pET Duet1 to generate plasmid pET Duet1-MiST. Sequence correction and the presence of the sortase motif were confirmed by means of Sanger sequencing. The *Anabaena variabilis* phenylalanine ammonia-lyase *(AvPAL)* sequence labeled with IC at its N-terminal was obtained by digestion with restriction enzymes of plasmid pET Duet1-muNS-Mi/2-IC-AvPAL previously obtained in the laboratory of the present inventors (non-published proprietary results) and inserted into the second polylinker of plasmid pET Duet1-MiST to obtain plasmid pET Duet1-MiST/2-IC-AvPAL. Sequence correction was confirmed by means of Sanger sequencing.

### MiST expression and purification

Competent BL21 bacteria (DE3) were transformed with plasmid pET Duet1-MiST and protein expression was induced by means of incubation at 37°C for 3 hours in the presence of 1 mM of IPTG. When total bacterial extracts were analyzed by means of SDS-PAGE, the presence of an intense protein band with the apparent MW of MiST was observed in the stained gel (Figure 1, lane 2) and it was not present in extracts of uninduced bacteria (Figure 1, lane 1). The published NS purification protocol (Barreiro-Piñeiro, N. et al. 2018, Scientific Reports 8, 16286) was then followed, resulting in the purified material shown in lane 3 of Figure 1, which indicates correct NS formation. Furthermore, the NS were observed under an optical microscope and were also characterized by means of DLS as particles with a mean diameter of 460 nm. To further confirm correct NS formation, uninduced and IPTG-induced bacteria were precipitated, fixed, and analyzed by means of TEM. The image in Figure 1b shows the presence of typical NS derived from muNS inside induced bacteria, which NS were not present in non-transformed bacteria (not shown).

### Sortase-mediated surface derivatization of MiST

The next step was to check whether the NS formed by MiST are capable of reacting with a compound containing polyG catalyzed by sortase. To that end, Sortase 5Δ (SiMPLe Protein Labeling Kit, BPS Bioscience) and the fluorescent substrate AZDye 488-Gly-Gly-Gly (Click Chemistry Tools, formula in Figure 2a) were used. In that sense, muNS-Mi NS or MiST NS purified at room temperature were incubated overnight with the substrate in the presence of sortase A. After incubation, both samples were dialyzed to remove excess substrate and then observed under a fluorescence microscope. The fluorescence shown by NS containing the sortase motif was obvious when observing them under the fluorescence microscope (Figure 2a, image MiST-IC), while no fluorescence was observed in muNS-Mi NS under the same conditions (Figure 3a. Image muNS-Mi), indicating the success of the reaction.

To eliminate the possibility that an unspecific non-covalent association was the cause of the fluorescent tagging observed, both NS preparations were subjected to SDS-PAGE and the unstained and unfixed gel was observed under UV light, showing a clear and intense fluorescent band corresponding to the molecular weight of MiST (Figure 2b, lane 4), while no fluorescent tagging appeared in the lane corresponding to muNS-Mi (Figure 2b, lane 3), nor in any case when sortase and the fluorescent substrate were absent (Figure 2b, lanes 1 and 2). The presence, the amount, and the identity of both proteins were also confirmed in parallel by means of a Western blot analysis using an anti-muNS antibody (Figure 2c). These results clearly demonstrate that the covalent binding between the fluorescent substrate and MiST catalyzed by sortase A was the cause of MiST tagging. Western blot analysis also shows the slight size difference between muNS-Mi and MiST, due to the addition of the sortase-target sequence (Figure 2c, compare lanes 1 and 2 or 3 and 4).

The effects of surface derivatization of MiST-NS with AZDye on its size and charge were then analyzed. DLS analysis showed that sortase-mediated modification caused an apparent NS size increase of about 19 nm. An increased negative charge measured by Z-potential was also observed. Both results conform perfectly to the characteristics of the added molecule (see formula in Figure 2).

**Table 1 - DLS analysis and comparison of MiST-IC not tagged or tagged with AZDye 488-Gly-Gly-Gly**

| | **Size (nm)** | **Pdl** | **Z-potential (mv)** |
|---|---|---|---|
| **MiST NS** | 459 ± 16 | 0.44 ± 0,10 | -27 ± 2 |
| **MiST NS+ AZDye 488-Gly-Gly-Gly** | 508 ± 21 | 0.50 ± 0,10 | -34 ± 2 |

### Co-purification of an external protein with MiST in NS

Having demonstrated that MiST is capable of forming NS correctly and that those NS also react with PolyG substrates through the added C-terminal sortase motif, the next step was to check whether this new construct was also capable of capturing IC-tagged proteins like the native muNS-Mi. In that sense, as mentioned in point 1, plasmid pET Duet1-MiST/IC-AvPAL was constructed. This plasmid drives the simultaneous expression of MiST (based on polylinker 1) and IC-AvPAL based on polylinker 2. After IPTG induction in BL21 bacteria (DE3), bands corresponding to the molecular weights of AvPAL and MiST were apparent in the stained gel (Figure 3, compare lanes 1 and 2). After following the inventors' published NS purification protocol (Barreiro-Piñeiro, N. et al. 2018, Scientific Reports 8, 16286), both proteins were apparently present in the NS, so they remained associated (Figure 3, lane 3). When purified NS were compared in parallel with non-modified muNS-Mi, both NS preparations were extremely similar (Figure 3, compare lanes 4 and 5), which indicates that the behavior of the new construct was identical to that of muNS-Mi not only in terms of inclusion formation, but also in terms of association with IC-tagged proteins, and it will work for all the described applications of the previously characterized IC-Tagging methodology. Again, the presence of sortase target sequence in MiST (Figure 3, lane 5) reduces its migration in acrylamide gel compared to non-modified muNS-Mi (Figure 3, lane 4). When the NS formed by MiST and containing AvPAL were observed under TEM, NS structures identical to those shown in Figure 1b were observed, as expected (not shown).

### Conclusions

- Despite the anticipated difficulties, a new modified version of muNS-Mi, referred to as MiST, carrying a sortase A target sequence at its C-terminal, has been developed.
- MiST is still capable of forming NS which capture IC-tagged proteins, thereby imitating a muNS-Mi in the IC-Tagging system.
- Furthermore, the NS formed by MiST can be further modified with compounds having an N-terminal polyglycine fraction (at least 3xGly) by means of incubation with sortase A.
- This new version of muNS significantly increases the versatility of the previously characterized IC-Tagging methodology, since virtually any compound can be added to the surface of the preformed spheres.

### Example 2

### Construction of plasmids pETDuet1.G4S-MiST and pETDuet1.G4S-MiST/IC-AvPAL

The muNS-Mi sequence was obtained by means of PCR amplification from plasmid pCIneo-muNS (488-635) (Brandariz-Nuñez et al., 2010 J. Virol. 84: 4289 - 4301) using 5'-CATGCCATGGCACCAGCCGTACTGCTGTC-3' (SEQ ID NO: 36) as a forward primer. The reverse primer was 5'-TTGCGGCCGCAATCAACCACCAGTCTCCGGGCAGAGAACCACCACCCAGATCATCCAC C-3' (SEQ ID NO: 37), which contains the sortase recognition motif sequence preceded by a G4S motif (SEQ ID NO: 29) and followed by a stop codon. This design permits the introduction of a G4S motif at the C-terminal end of the muNS-Mi sequence which functions as a flexible spacer before the LPETG motif (SEQ ID NO: 23). The amplified product was inserted into the first polylinker of plasmid pETDuet1 to generate plasmid pETDuet1.G4S-MiST. Sequence correction was confirmed by means of Sanger sequencing. The *Anabaena variabilis* phenylalanine ammonia-lyase (AvPAL) sequence labeled with IC at its N-terminal end was obtained by means of digestion with restriction enzymes Ndel and Bglll of plasmid pETDuet1.muNS-Mi 2.IC-AvPAL previously obtained in the laboratory of the present inventors (non-published results) and inserted into the second polylinker of plasmid pETDuet1.G4S-MiST to obtain plasmid pETDuet1. G4S-MiST/IC-AvPAL. Sequence correction was confirmed by means of Sanger sequencing.

Figures 5 to 8 describe the expression and purification both for the "normal" version (MiST) and for the version with the linker (G4S-MiST), as well as for the respective versions loaded with the IC-tagged AvPAL enzyme. Methods for the expression and purification of the new versions of G4S-MiST are the same as those for MiST.

Table 2 shows the values of the size (nm), Pdl, and Z-potential (mV) of the different NS before and after reacting with the fluorescent compound in the presence of SrtA. Data is depicted as mean ± standard deviation; n=3. Significant differences for α =0.05 are indicated with asterisks. GraphPad Prism was used to trace the data and for statistical analyses.

**Table 2: DLS and Z-potential analysis of functionalized NS**

| | Size (nm) | | Pdl | | Z-potential (mV) | |
|---|---|---|---|---|---|---|
| NS type | Control | +AZDye +SrtA | Control | +AZDye +SrtA | Control | +AZDye +SrtA |
| muNS-Mi | 491±31 | 444±209 | 0,22±0,16 | 0,65±0,14 | -26±1 | -28+1 |
| MiST | 342±22 | 575±61 | 0.40±0.04 | 0.23±0.19 | -23±2 | -31±2 |
| G4S-MiST | 299±89 | 739±230 | 0.32±0.03 | 0.33±0.15 | -23±1 | -29±3 |
| muNS-Mi/ IC-AvPAL | 679±46 | 670±101 | 0.10±0.10 | 0.59±0.36 | -29±1 | -26±1 |
| MIST/ IC-AvPAL | 853±39 | 932±141 | 0.37±0.35 | 0.46±0.47 | -25±1 | -30±2 |
| G4S-MiST/ IC-AvPAL | 721±64 | 888±153 | 0.18±0.14 | 0.75±0.43 | -27±2 | -30± |

After demonstrating that MiST NS can be functionalized with small substrates, the possibility of aggregating a whole protein covalently mediated by sortase was tested. To that end, a modified eGFP protein containing a N-terminal polyglycine tail and incubated with MiST NS that are empty or loaded with AvPAL in the presence (+SrtA ) or absence of sortase A was used. The images of Figure 9 show that both MiST and G4S-MiST are labeled with GFP only after incubation with sortase A. Moreover, the presence of the linker does not show any significant improvement in tagging.

NS samples shown in Figure 9 were subjected to electrophoresis under semi-denaturing conditions to retain the fluorescence capacity of GFP. As shown in Figure 10, MiST (lanes 2 and 3) and G4S-MiST (lanes 4 and 5), either alone (2 and 4) or loaded with AvPAL (3 and 5), bind covalently to glycine-containing GFP after reaction with sortase, since the appearance of additional bands corresponding to the size of the eGFP-MiST binding product can be observed. In the case of the versions with encapsulated enzyme, a second additional band of a larger size (which may correspond to a type of dimer resulting from the reaction) was also observed.

To complete the analysis, new reactions were performed with NS loaded with enzyme to be analyzed this time by means of complete SDS-PAGE denaturation. The Coomassie-stained gel shown in Figure 11 clearly shows that, after reacting with sortase A in the presence of glycine-containing GFP (lanes 3 and 4), both MiST (lane 3) and G4S-MiST (lane 4) produce a band corresponding to the molecular weight of the fusion between GFP and MiST. The presence of the G4S linker does not show any apparent improvement in the reaction.

### EXAMPLE 3

Since it has been demonstrated that MiST nanospheres (NS) can be functionalized with whole proteins (such as GFP) which contain an oligo-Gly tail at their N-terminal end, the inventors attempted to construct a new MiST variant that would be of help in cases where it is not possible or suitable to modify the N-terminal end of the protein to be added with oligo-Gly, for example, to suitably orient a binding surface, to prevent folding obstacles, etc. For such cases, it might be better to carry out the opposite strategy, i.e., to add an oligo-Gly sequence to the N-terminal end of muNS-Mi that could react with sortase motif-carrying molecules in the presence of sortase A. This strategy is risky because the success in constructing a correctly ordered inclusion of different versions that have been tested with muNS depends largely on the sequence added at the N-terminal end, which often resulted in amorphous, disordered, and useless aggregates (Brandariz-Nuñez et al., 2010 J. Virol. 84: 4289 - 4301). To achieve this objective, a tract of three glycines was initially added to the N-terminal end of muNS-Mi just after the initial methionine residue to create the 3G-MiST fusion protein. Furthermore, a construct carrying a TEV protease motif (ENLYFQG) at the N-terminal end, followed by two additional Gly, was also designed. As the TEV protease cleaves between Q and G, after digestion of the ENLYFQG sequence, the N-terminal Gly 3 required for the sortase reaction will be available at the N-terminal end of the construct. BL21 bacteria were transformed with the resulting plasmids pDuet1-3G-MiSt and pDuet1-TEV-3G-MiST, and protein extracts were compared before and after (Figure 12, lanes 1 and 2, respectively) IPTG induction.

The expression of both constructs was clear in the gels analyzed in Figure 12. Although the expression of the version containing the TEV sequence is better than that of 3 Gly, the characterization of both versions was performed. First, the purification thereof was carried out by performing the protocol described above for the nanospheres produced with IC-Tagging and MiSt-IC. As shown in Figure 13A, both versions were easily purified with the standard protocol, which indicates a behavior similar to the muNS-Mi or MiST versions. Furthermore, both versions were also susceptible to the modification with sortase, since upon incubating same with the fluorescent substrate 5-FAM-LPETG (SEQ ID NO: 23) (LPETG being the sortase motif), they were covalently labeled with the fluorescent compound, only when sortase A was included in the reaction. Furthermore, for TEV-3G-MiST, a TEV digestion must be performed before sortase reaction to leave the 3G motif available at its N-terminal end, as inferred from the results shown in Figure 13B.

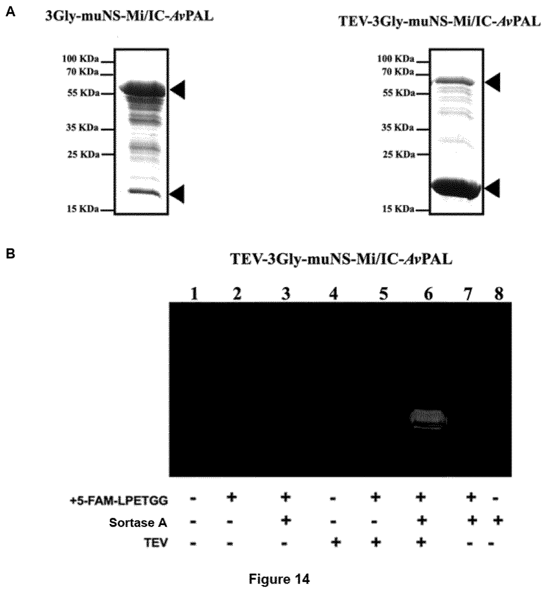

The same results were obtained with 3G-MiST and TEV-3G-MiST spheres loaded with an exogenous protein (AvPAL), as demonstrated in Figure 14. Again, the difference is that 3G-MiST has a lower expression than TEV-3G-MiST, but both capture IC-tagged proteins, are purified with the inventors' published protocol, and react with ligands having the sortase motif through the mediation of sortase A.

## Claims

1. A fusion protein comprising the following components:
(i) a polypeptide comprising a sequence selected from the group consisting of:
- sequence 448-635 (SEQ **ID** NO: 1) of the avian *Orthoreovirus* muNS protein,
- sequence 518-721 (SEQ **ID** NO: 2) of the mammalian *Orthoreovirus* muNS protein, and
- a functionally equivalent variant of any of the above with the capacity to form nanospheres and/or microspheres, and
(ii) a polypeptide selected from:
(a) a polypeptide comprising the sortase recognition motif or the residual part of a sortase recognition motif generated after a sortase-mediated reaction, wherein component (ii) is fused to the carboxyl end of component (i), or
(b) a polypeptide comprising the sortase acceptor motif, wherein component (ii) is fused to the amino end of component (i).

2. The fusion protein according to claim 1, wherein the sortase recognition motif is amino acid sequence LPXTG (SEQ **ID** NO: 22), wherein X is any amino acid.

3. The fusion protein according to claim 2, wherein the sortase recognition motif is amino acid sequence LPETG (SEQ **ID** NO: 23).

4. The fusion protein according to any of claims 1 to 3, wherein the fusion protein comprising component (ii)(a) further comprises a component (iii) fused to the amino end of component (i), where component (iii) is a first polypeptide of interest.

5. The fusion protein according to claim 4, wherein component (iii) is fused to component (i) by a protease recognition sequence.

6. The fusion protein according to claim 5, wherein the protease recognition sequence is an enterokinase recognition sequence or a factor Xa recognition sequence.

7. The fusion protein according to any of claims 4 to 6, wherein component (iii) comprises a secretory pathway signal peptide.

8. The fusion protein according to claim 7, wherein the signal peptide comprises the sequence SEQ ID NO: 10.

9. The fusion protein according to any of claims 4 to 8, wherein component (iii) comprises a peptide to facilitate purification thereof.

10. The fusion protein according to any of claims 1 to 9, wherein the fusion protein comprising component (ii)(a) further comprises a component of interest covalently bound to the residual part of the sortase recognition motif generated after a sortase-mediated reaction.

11. The fusion protein according to claim 10, wherein the component of interest is selected from a group consisting of: toll-like receptor (TLR) ligands, monophosphoryl lipid A (MPL A), oligonucleotides selected from CpG islands and polyinosinic:polycytidylic acid (poly(I:C) - CAS number 24939-03-5), saponin, lipid molecules, coating molecules, oligosaccharides, antibodies, nanoantibodies, affitins, viral antigen, a bacterial antigen, a fungal antigen, an allergen, a cell-penetrating peptide, an affibody, or an environmental antigen, a tumor antigen, wherein the component of interest is **characterized by** comprising an oligoglycine sequence of at least 3 glycines.

12. The fusion protein according to any of claims 1 to 11, wherein component (i) and component (ii)(a) are covalently bound through a flexible peptide linker.

13. The fusion protein according to claim 12, wherein the flexible peptide linker comprises an amino acid sequence according to SEQ ID NO: 29 (GGGGS).

14. The fusion protein according to claim 1, wherein the sortase acceptor motif comprises an amino acid sequence with at least between 1 and 20 non-polar amino acids.

15. The fusion protein according to claim 14, wherein the non-polar amino acid sequence comprises a plurality of glycines (G) or alanines (A).

16. The fusion protein according to claim 15, wherein the amino acid sequence is the sequence G[G]ₙ or A[A]ₙ, wherein n = 0-2.

17. The fusion protein according to claim 16, wherein the amino acid sequence is selected from a group consisting of: G, A, GG, AA, GGG, and AAA.

18. The fusion protein according to claim 17, wherein the amino acid sequence consists of sequence GGG.

19. The fusion protein according to any of claims 1 and 14 to 17, wherein the fusion protein further comprises a protease recognition sequence covalently bound to the amine end of the sortase acceptor motif.

20. The fusion protein according to claim 19, wherein the protease recognition sequence is an enterokinase recognition sequence or a factor Xa recognition sequence.

21. The fusion protein according to any of claims 1 and 14 to 20, wherein the fusion protein comprising component (ii)(b) further comprises a component of interest covalently bound to the sortase acceptor motif.

22. The fusion protein according to claim 21, wherein the component of interest is selected from a group consisting of: toll-like receptor (TLR) ligands, monophosphoryl lipid A (MPL A), oligonucleotides selected from CpG islands and polyinosinic:polycytidylic acid (poly(I:C) - CAS number 24939-03-5), saponin, lipid molecules, coating molecules, oligosaccharides, antibodies, nanoantibodies, affitins, viral antigen, a bacterial antigen, a fungal antigen, an allergen, a cell-penetrating peptide, an affibody, or an environmental antigen, a tumor antigen, wherein the component of interest is **characterized by** comprising a sortase recognition sequence or the residual part of a sortase recognition motif generated after a sortase-mediated reaction.

23. A nanosphere or a microsphere comprising a fusion protein according to any of claims 1 to 22.

24. The nanosphere or microsphere according to claim 23, further comprising a second fusion protein comprising the following components:
(a) a polypeptide selected from the group consisting of:
- a polypeptide comprising sequence 477-542 (SEQ ID NO: 25) of the avian *Orthoreovirus* muNS protein,
- a polypeptide comprising sequence 561-622 (SEQ ID NO: 26) of the mammalian *Orthoreovirus* muNS protein,
- a functionally equivalent variant of any of the above which retains the capacity to be incorporated into nanospheres, and
(b) a second polypeptide of interest.

25. The nanosphere according to claim 23 or 24, wherein the nanosphere has a size of between 300 nm and 550 nm.

26. The microsphere according to claim 23 or 24, wherein the microsphere has a size of between 0.55 µm and 4 µm.

27. The nanosphere or microsphere according to any of claims 23 to 26, wherein the fusion protein comprising component (ii)(a) further comprises a component of interest covalently bound to the residual part of the sortase recognition motif generated after a sortase-mediated reaction.

28. The nanosphere or microsphere according to any of claims 22 to 25, wherein the fusion protein comprising component (ii)(b) further comprises a component of interest covalently bound to the sortase acceptor motif.

29. The nanosphere or microsphere according to claim 27 or 28, wherein the component of interest is selected from the group consisting of:
- toll-like receptor (TLR) ligands;
- monophosphoryl lipid A (MPL A);
- oligonucleotides selected from CpG islands and polyinosinic:polycytidylic acid (poly(I:C) - CAS number 24939-03-5);
- saponin;
- lipid molecules;
- coating molecules selected from a group consisting of: polyglycerols, polyethylene glycol, chitosans, poly(oxazolines) (POX), poly(hydroxypropyl methacrylate) (PHPMA), poly(2-hydroxyethyl methacrylate) (PHEMA), poly(N-(2-hydroxypropyl)methacrylamide) (HPMA), polyvinylpyrrolidone (PVP), poly(N,N-dimethylacrylamide) (PDMA), and poly(N-acryloylmorpholine) (PAcM);
- oligosaccharides;
- antibodies;
- nanoantibodies;
- affitins;
- viral antigen;
- bacterial antigen;
- fungal antigen;
- allergen;
- cell-penetrating peptides;
- affibody;
- environmental antigen; and
- a tumor antigen.

30. The nanosphere or microsphere according to any of claims 23 to 29, wherein the first polypeptide of interest, and/or the second polypeptide of interest, and/or the component of interest is the glucose-6-phosphatase 2 protein (IGRP).

31. A polynucleotide encoding a fusion protein according to any of claims 1 to 22.

32. An expression cassette comprising a polynucleotide according to claim 31.

33. A vector comprising a polynucleotide according to claim 31 or an expression cassette according to claim 32.

34. A vector according to claim 33, further comprising a second polynucleotide encoding a polypeptide selected from the group consisting of:
- a polypeptide comprising sequence 477-542 (SEQ ID NO: 25) of the avian *Orthoreovirus* muNS protein,
- a polypeptide comprising sequence 561-622 (SEQ ID NO: 26) of the mammalian *Orthoreovirus* muNS protein,
- a functionally equivalent variant of any of the above which retains the capacity to be incorporated into nanospheres.

35. A vector composition comprising the vector according to claim 34 and a second vector comprising a second polynucleotide encoding a polypeptide selected from the group consisting of:
- a polypeptide comprising sequence 477-542 (SEQ ID NO: 25) of the avian *Orthoreovirus* muNS protein,
- a polypeptide comprising sequence 561-622 (SEQ ID NO: 26) of the mammalian *Orthoreovirus* muNS protein,
- a functionally equivalent variant of any of the above which retains the capacity to be incorporated into nanospheres.

36. The vector according to claim 33 or 34 or the vector composition according to claim 35, wherein the polynucleotide according to claim 31 or the expression cassette according to claim 32 is operatively bound to a first promoter and the second polynucleotide is operatively bound to a second promoter.

37. The vector or vector composition according to claim 36, wherein the first and second promoters are the same promoter or are different promoters.

38. The vector or vector composition according to any of claims 34 to 37, wherein said vector(s) is/are (an) bacterial expression vector(s).

39. A cell comprising a fusion protein according to any of claims 1 to 22, a polynucleotide according to claim 31, an expression cassette according to claim 32, a vector, or a vector composition according to any of claims 33 to 38.

40. A method for producing a fusion protein according to any of claims 1 to 22, which comprises:
(a) expressing in a cell a first polynucleotide encoding said fusion protein,
(b) subjecting said cell to conditions suitable for the formation of nanospheres or microspheres, and
(c) concentrating the nanospheres or microspheres.

41. The method according to claim 39, which further comprises purifying the fusion protein by separating it from the nanospheres or microspheres.

42. The method according to claim 40 or 41, wherein if the fusion protein comprises component (ii)(b) and a protease recognition sequence fused to the amino end of the sortase acceptor motif, the method further comprises contacting the fusion protein with a protease specific for the protease recognition sequence that is fused to the amino end of the sortase acceptor sequence, under conditions suitable for the proteolysis of said recognition sequence, with the subsequent separation of the protease recognition sequence of the fusion protein.

43. The method according to any of claims 40 to 42, wherein the cell is a bacterial cell or a eukaryotic cell, wherein if it is a bacterial cell, nanospheres are formed in step (b), and if it is a eukaryotic cell, microspheres are formed in step (b).

44. A method for producing a protein which comprises:
(a) expressing in a cell a first polynucleotide encoding a fusion protein according to claims 1 to 22 and a second polynucleotide encoding a second fusion protein comprising components:
(i) a polypeptide selected from the group consisting of:
- a polypeptide comprising sequence 477-542 (SEQ ID NO: 25) of the avian *Orthoreovirus* muNS protein,
- a polypeptide comprising sequence 518-721 (SEQ ID NO: 26) of the mammalian *Orthoreovirus* muNS protein,
- a functionally equivalent variant of any of the above which retains the capacity to be incorporated into nanospheres, and
(ii) a second polypeptide of interest,
wherein component (ii) is the protein that is produced;
(b) subjecting said cell to conditions suitable for the formation of nanospheres or microspheres, and
(c) concentrating the nanospheres or microspheres.

45. The method according to claim 44, which further comprises purifying the fusion protein by separating it from the nanospheres or microspheres.

46. The method according to claim 44 or 45, wherein the cell is a bacterial cell or a eukaryotic cell, wherein if it is a bacterial cell, nanospheres are formed in step (b), and if it is a eukaryotic cell, microspheres are formed in step (b).

47. The method according to any of claims 44 to 46, wherein the protein is the IGRP protein.

48. A method for producing a first polypeptide of interest, wherein the method comprises the following steps:
(a) producing the fusion protein by means of the method according to any of claims 42 to 45, wherein the fusion protein comprises:
- component (ii)(a) and a component (iii) fused to the amino end of component (i), wherein component (iii) is the polypeptide of interest, and
- a protease recognition sequence between its components (i) and (iii),
(b) subjecting the nanospheres and/or microspheres to conditions leading to the disintegration thereof, causing the separation of the fusion protein and the nanospheres and/or microspheres,
(c) contacting the product resulting from step (b) with a protease specific for the recognition sequence connecting components (i) and (iii) of the fusion protein under conditions suitable for the proteolysis of said fusion protein, with the subsequent separation of components (i) and (iii) of the fusion protein,
(d) subjecting the product of step (c) to conditions suitable for the formation of the nanospheres and/or microspheres, and
(e) separating the nanospheres and/or microspheres from component (iii).

49. A protein or polypeptide obtainable according to the method of any of claims 44 to 48.

50. A method for producing a nanosphere or microsphere according to claims 23 to 30, comprising the steps of:
(a) producing the fusion protein by means of the method according to claim 39 or 41, and
(b) subjecting said cell to conditions suitable for the formation of nanospheres or microspheres.

51. The method according to claim 50, further comprising the step of incubating the nanospheres and/or microspheres in the presence of a sortase that recognizes the sortase recognition motif of component (ii)(a) of the fusion protein and a substrate comprising a sortase acceptor motif, obtaining nanospheres and/or microspheres comprising the substrate that comprises the tag recognized by the sortase.

52. The method according to claim 50, further comprising the step of incubating the nanospheres and/or microspheres in the presence of a sortase that recognizes the sortase acceptor motif in component (ii)(b) of the fusion protein and a substrate comprising a sortase recognition motif, obtaining nanospheres and/or microspheres comprising the substrate that comprises the sortase recognition motif.

53. The method according to any of claims 50 to 52, wherein the cell is a bacterial cell or a eukaryotic cell, wherein if it is a bacterial cell, nanospheres are formed in step (b), and if it is a eukaryotic cell, microspheres are formed in step (b).

54. The method according to any of claims 50 to 53, wherein the nanospheres or microspheres are purified between step (b) and step (c).

55. The method according to any of claims 50 to 54, wherein the sortase is sortase A, the recognition motif is the sequence SEQ ID NO: 22 or SEQ ID NO: 23, and the sortase acceptor motif is a polyglycine sequence, preferably sequence GGG.

56. A pharmaceutical composition or immunogenic composition comprising the nanosphere and/or microsphere according to any of claims 23 to 30 and a pharmaceutically acceptable excipient.

57. The nanospheres and/or microspheres according to any of claims 23 to 30 for use in medicine.

58. The nanospheres and/or microspheres according to any of claims 23 to 30 or the immunogenic composition according to claim 56 for use in the treatment and/or prevention of bluetongue, wherein the fusion proteins comprised in the nanosphere are **characterized by** comprising at least one of:
- the bluetongue virus 4 (BTV) outer capsid protein 2 (VP2);
- the bluetongue virus 4 (BTV) core protein 7 (VP7);
- the bluetongue virus 4 (BTV) non-structural protein 1 (NS1);
- a protein with a sequence functionally equivalent to any of the above.

59. The nanospheres and/or microspheres according to any of claims 23 to 30 or the immunogenic composition according to claim 56 for use in the treatment and/or prevention of African horse sickness, wherein the fusion proteins comprised in the nanosphere are **characterized by** comprising at least one of:
- the African horse sickness virus (AHSV) non-structural protein 1 (NS1);
- a protein with a sequence functionally equivalent to any of the above.

60. The nanospheres and/or microspheres according to any of claims 23 to 30 or the pharmaceutical composition according to claim 56 for use in the treatment and/or prevention of type 1 diabetes, wherein the fusion protein comprised in the nanosphere is **characterized by** comprising the glucose-6-phosphatase 2 protein (IGRP) according to the sequence with accession number Q9NQR9 in the UniProt database, entry dated August 3, 2022, sequence version 1.

61. The nanospheres and/or microspheres for use according to claim 60, wherein the type 1 diabetes is latent autoimmune diabetes in adults.

62. The nanospheres and/or microspheres for use according to any of claims 58 to 61, wherein the administration of the nanosphere is by subcutaneous or intravenous route.

63. The nanospheres and/or microspheres for use according to any of claims 58 to 62, wherein the nanosphere and/or microsphere is administered in the absence of an adjuvant.

64. A method for inducing type 1 diabetes in an animal model, which comprises administering to an animal an effective amount of the nanosphere according to claim 30.

65. The method according to claim 64, wherein the administration is by subcutaneous or intramuscular route.

66. The method according to any of claims 64 or 65, wherein the nanosphere is administered together with an adjuvant.
